(19)

**Europäisches Patentamt**
**European Patent Office**
**Office européen des brevets**

(11) **EP 3 415 195 A1**

(12) **EUROPEAN PATENT APPLICATION**

(43) Date of publication:
**19.12.2018 Bulletin 2018/51**

(51) Int Cl.:
*A61N 1/05* (2006.01)  *H01B 1/02* (2006.01)

(21) Application number: **17175928.5**

(22) Date of filing: **14.06.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **Heraeus Deutschland GmbH & Co. KG**
  **63450 Hanau (DE)**
• **Saes Smart Materials, Inc.**
  **New Hartford, NY 13413 (US)**
• **Heraeus Materials Singapore Pte. Ltd.**
  **569881 Singapore (SG)**
• **Heraeus Medical Components, LLC**
  **St. Paul, MN 55127 (US)**

(72) Inventors:
• **RICHTER, René**
  **64832 Babenhausen (DE)**

• **GEBERT, Jörg-Martin**
  **76227 Karlsruhe (DE)**
• **SPECHT, Heiko**
  **63456 Hanau (DE)**
• **SCZERZENIE, Francis E.**
  **Lake Pleasant, NY New York 12108 (US)**
• **MANJERI, Radhakrishnan M.**
  **New Hartford, NY New York 13413-2250 (US)**
• **YIN, Weimin**
  **Ridgefield, CT Connecticut 06877 (US)**
• **GOLAGANI VENKATA KANAKA, Sai Srikanth**
  **730586 Singapore (SG)**
• **LARK, Larry**
  **Saint Paul, MN Minnesota 55116 (US)**

(74) Representative: **Maiwald Patent- und
Rechtsanwaltsgesellschaft mbH
Elisenhof
Elisenstraße 3
80335 München (DE)**

(54) **A METHOD FOR MANUFACTURING A CABLE**

(57)    The invention generally relates to a method for manufacturing a cable, a cable manufactured by such method, and a medical device comprising such cable. The cable comprises an alloy comprising Cr, Ni, Mo and Co, preferably with tightly controlled levels of impurities. The method for manufacturing the cable comprises the steps of providing several raw wires made of a wire material, drawing the raw wires into wires, coiling the wires into a cable, and heat treating the cable. The wire material comprises an alloy comprising the following alloy components:

a) Cr in the range from about 10 to about 30 wt. %;
b) Ni in the range from about 20 to about 50 wt. %;
c) Mo in the range from about 2 to about 20 wt. %;
d) Co in the range from about 10 to about 50 wt. %.

The A1 content of the Cr, Ni, Mo and Co alloy is less than about 0.01 wt. % and each wt. % is based on the total weight of the alloy.

Fig. 14

## Description

### Field of the invention

The invention generally relates to a method for manufacturing a cable, a cable manufactured by such method, and a medical device comprising such cable. The cable comprises an alloy comprising Cr, Ni, Mo and Co, preferably with tightly controlled levels of impurities.

### Background of the invention

Much investigation in recent years has been directed to a search for new high performance alloys, particularly for medical applications where a very high value is placed on reliability and materials are required which exhibit a low failure rate even over a long time period.

Cardiac Pacemakers, Implantable Cardioverter Defibrillation Devices and Cardiac Resynchronisation Devices are applications where reliability is particularly important, especially in terms of resistance to physical fatigue and to chemical corrosion. Invasive surgery is required to implant a pacemaker into the body or remove or replace parts, and it is highly desirable for the individual components of the pacemaker to have a long working life in order to reduce the requirement for surgical intervention. Furthermore, it is desirable for the working life to have a low variance. In a heart pacemaker, one component which is exposed to a particularly high amount of stress during normal operation is the so called lead which connects the implantable pulse generator to the heart tissue. A flexible lead is required in order to connect the implantable pulse generator to the heart tissue without imposing undue physical stress on the heart and the lead flexes during normal operation, typically repetitively with a frequency on the order of that of a human heart beat. A high resistance to fatigue is therefore required in the lead in order to withstand frequent physical stress over a long period of time. A high resistance of the lead to corrosion is important not only in terms of the lifetime of the component, but also in terms of reducing toxicity to the body.

WO 2005026399 A1 discusses an approach to improving the properties of an alloy by reducing the content of titanium nitride and mixed metal carbonitride.

US 2005/0051243 A1 focuses on alloys with a reduced content of nitrogen.

The fatigue resistance may still be improved.

### Summary of the invention

Hence, there may be a need to provide an improved manufacturing method for a cable, which in particular allows an improved fatigue resistance of the cable.

The problem of the present invention is solved by the subject-matters of the independent claims, wherein further embodiments are incorporated in the dependent claims. It should be noted that the aspects of the invention described in the following apply also to the method for manufacturing a cable, the cable manufactured by such method, and the medical device comprising such cable.

According to the present invention, a method for manufacturing a cable is presented. The method for manufacturing the cable comprises the following steps, not necessarily in this order:

- providing several raw wires made of a wire material,
- drawing the raw wires into wires,
- coiling the wires into a cable, and
- heat treating the cable.

The wire material comprises an alloy comprising the following alloy components:

    a) Cr in the range from about 10 to about 30 wt. %;
    b) Ni in the range from about 20 to about 50 wt. %;
    c) Mo in the range from about 2 to about 20 wt. %;
    d) Co in the range from about 10 to about 50 wt. %.

The A1 content of the Cr, Ni, Mo and Co alloy is less than about 0.01 wt. % and each wt. % is based on the total weight of the alloy.

The combination of drawing, coiling and heat treating according to the present invention results in a deformation and heat treatment process, which is particular beneficial for a fatigue resistance of the cable.

A raw wire or wire may be configured to bear mechanical loads or electricity and/or telecommunications signals.

The raw wire or wire may be flexible and may be circular in cross-section or square, hexagonal, flattened, rectangular or the like. The raw wire or wire may be a semifinished or finished product used for medical applications such as coils or strands used for e.g. cardiac rhythm management.

A difference between a raw wire and a wire according to claim 1 is that a wire is a raw wire after drawing or a drawn raw wire. Drawing and here in particular cold drawing is a method to reduce a diameter or to increase a length of the sample. A diameter of the wire may be in a range of 10 to 500 $\mu$m.

At least two wires can be joined to form a cable. A cable is therefore a combination of least two, several or a plurality of wires. The wires may be coiled, wound or stranded to form the cable. The cable may have various configurations like e.g. 1x7, 7x7, 1x19, 7x19 and the like. The cable may be configured for a medical application.

The wire material comprises a Cr, Ni, Mo and Co alloy, which may be an alloy having improved resistance to physical fatigue, high corrosion resistance and/or an ability to be drawn into a particular thin wire. In an example, the Cr, Ni, Mo and Co components are major constituents of the Cr, Ni, Mo and Co alloy with at least about 95 wt. % of the alloy being Cr, Ni, Mo and Co. Details in view of the alloy are provided further below. The Cr, Ni, Mo and Co alloy may be MP35N, MP35NLT and the like.

A cold work percentage (%CW) is used to express a degree of plastic deformation and defined as

$$\%CW = \left( \frac{A_0 - A_d}{A_0} \right) \times 100$$

wherein $A_0$ is an initial or original cross sectional area and $A_d$ is an area after deformation. In an example, the raw wires are drawn with a cold work percentage in a range of 98 and 99 %. In another example, the raw wires are drawn with a cold work percentage in a range of 80 and 86 %. Such rather high deformation or cold work percentages may lead to a cable with a very high YS/UTS ratio and/or a cable with very high strength. These benefits are based on the high amount of cold work percentage, which leads to a formation of nanograins in a microstructure of the wire. The high amount of cold work percentage may also lead to a heavily dislocated microstructure, which leads to a considerably improved fatigue resistance of the cable.

In an example, the heat treatment of the cable is in a temperature range of 480 to 750 °C. In an example, the heat treatment of the cable is with a dwell time of 5 to 8 seconds and preferably 5 to 6 seconds. Such rather low temperatures may also lead to an improved fatigue resistance of the cable.

In particular a combination of a cold work percentage in a range of 98 and 99 % and a heat treatment of the cable is in a temperature range of 480 to 750 °C may result in cables with high YS/UTS ratios e.g. in a range of 1.0-1.1 and/or a low loss of ductility in the cables. A combination of a cold work percentage in a range of 80 and 86 % and a heat treatment of the cable is in a temperature range of 480 to 750 °C may result in cables with lower YS/UTS ratios e.g. in a range of 0.8-0.9.

In an example, the heat treatment of the cable is a stress relief treatment in a temperature range of 480 to 580°C and preferably 550 to 580°C to improve a low cycle fatigue performance of the cable. A dwell time may be in a range of 5 to 10 seconds. The LCF performance of the cable is improved when compared to e.g. conventional cables and/or cables subjected to a heat treatment with a temperature range of 780 to 880°C. In another example, the heat treatment of the cable is an annealing in a temperature range of 780 to 810 °C to improve a high cycle fatigue performance of the cable. A dwell time may be in a range of 5 to 10 seconds. An aging effect is significantly different for cables when compared to wires, as there is an improvement in ductility of the wire and a reduction in strength of the cable when stress is relieved at higher temperatures when compared to drawn wires. Measured values are shown further below.

The manufacturing method according to the present invention may comprise more drawing and/or heat treating steps. For example, an initial drawing and/or an initial heat treating step may be arranged before above explained drawing and heat treating steps of claim 1.

In an example, the manufacturing method for the cable further comprises an initial drawing of a raw material into the raw wires. The initial drawing may also relate to any prior material, intermediate wire before being drawn to a raw wire or the like. This initial drawing may be arranged before above explained drawing and heat treating steps of claim 1. In this example, the raw material may be drawn with a cold work percentage in a range of 95 and 96 %.

In an example, the manufacturing method for the cable further comprises an initial heat treatment of the raw wires before the drawing of the raw wires into the wires. The initial heat treatment may also relate to any prior material, intermediate wire before being drawn to a raw wire or the like. This initial heat treating may be arranged before above explained drawing and heat treating steps of claim 1. The initial heat treating may also be arranged after the above explained initial drawing step. In this example, the initial heat treatment of the raw wires may be an annealing in a temperature range of 875 to 1100 °C. The temperature range may also be between 875 and 950 °C or between 950 and 1100°C. A dwell time of the initial heat treating may be in a range of 2 to 15 seconds and preferably 5 to 8 seconds.

The wire may be biocompatible. The wire may be made of the Cr, Ni, Mo and Co alloy only. The wire may also be a composite wire comprising a part made of the Cr, Ni, Mo and Co alloy and another part made of an additional, different and preferably metallic material. The other part may be made of a metal or another alloy. In other words, the wire material may comprise an additional material different to the Cr, Ni, Mo and Co alloy. In an example, the additional material comprises at least one of a group of Silver, Platinum, Tantalum, Gold, Copper and alloys thereof. The additional material may comprise at least one of a group of: Platinum, a Platinum based alloy, a Platinum-Iridium alloy, a Platinum-Tungsten alloy, Gold, a Gold alloy, Tantalum, Titanium, a Titanium-Molybdenum alloy, and a Titanium Aluminum Vanadium alloy. The additional material may increase an electrical conductivity of the cable.

In an example, the additional material forms a core and the Cr, Ni, Mo and Co alloy forms a shell around the core when the wire is seen in a cross section. In another example, the Cr, Ni, Mo and Co alloy forms a core and the additional material forms a shell around the core when the wire is seen in a cross section. In both cases, when seen in a cross section, a filling ratio between an area of the core (filler) and an area of the shell (hollow tube) may be between 5 and 75 percent and preferably between 15 to 41 percent. Of course, the wire may still comprise at least a second additional material as e.g. outermost layer and coating or inner core.

According to the present invention, also a cable comprising drawn, coiled and heat treated wires as described above as a lead is presented. Assuming a wire diameter of 25 to 27 $\mu$m in a cable drawn, coiled and heat treated according to claim 1 and a 1x7 cable configuration, a total of 7 wires in the cable make up to an outer diameter of 76 to 78 $\mu$m. In case of a 7x7 cable configuration, a total of 49 wires in the cable make up to an outer diameter of 270 to 275 $\mu$m, and in case of a 1x19 cable configuration, a total of 19 wires in the cable make up to an outer diameter of 125 to 128 $\mu$m.

According to the present invention, also a medical device comprising a cable as described above as a lead is presented. The medical device may be a pacemaker, an implantable cardioverter defibrillator, a cardiac resyncronisation device, a cardiac rhythmic management device, a neuromodulation device, a neurostimulation device, a spinal cord stimulation device, a (deep) brain stimulation device, a cochlea implant or any other implantable stimulation device comprising a composite wire as described above as a lead.

The Cr, Ni, Mo and Co alloy may be an alloy, which has improved resistance to physical fatigue, a high corrosion resistance, and/or which can be drawn into a thin wire, preferably less than about 50 $\mu$m. The wire according to the invention may be a wire having comparable tensile properties to known wires, but for which the proportion of outlying failures in fatigue resistance is reduced.

A contribution to achieving at least one of the above described objects is made by the following embodiments of the Cr, Ni, Mo and Co alloy (in the following "alloy").

|1| An alloy comprising the following alloy components:

a) Cr in the range from about 10 to about 30 wt. %, preferably in the range from about 15 to about 25 wt. %, more preferably in the range from about 19 to about 21 wt. %;
b) Ni in the range from about 20 to about 50 wt. %, preferably in the range from about 30 to about 45 wt. %, more preferably in the range from about 33 to about 37 wt. %;
c) Mo in the range from about 2 to about 20 wt. %, preferably in the range from about 5 to about 15 wt. %, more preferably in the range from about 9 to about 10.5 wt. %;
d) Co in the range from about 10 to about 50 wt. %, preferably in the range from about 20 to about 40 wt. %, more preferably in the range from about 33 to about 37 wt. %;

wherein the Al content of the alloy is less than about 0.01 wt. %, preferably less than about 0.005 wt. %, more preferably less than about 0.001 wt. %;
wherein each wt. % is based on the total weight of the alloy.

|2| The alloy according to embodiment |1|, wherein the content of Mg is less than about 0.005 wt. %, preferably less than about 0.0001 wt. %, more preferably less than about 0.00001 wt. %, based on the total weight of the alloy.

|3| The alloy according to embodiment |1| or |2|, wherein the content of Ca is less than about 0.005 wt. %, preferably less than about 0.0001 wt. % more preferably less than about 0.00001 wt. %, based on the total weight of the alloy.

|4| The alloy according to any of the preceding embodiments, wherein the content of Ce is less than about 0.005 wt. %, preferably less than about 0.0001 wt. % more preferably less than about 0.00001 wt. %, based on the total weight of the alloy.

|5| The alloy according to any of the preceding embodiments, wherein the content of Ti is less than about 0.1 wt.

%, preferably less than about 0.01 wt. % more preferably less than about 0.001 wt. %, further more preferably less than about 0.0005 wt. %, based on the total weight of the alloy.

|6| The alloy according to any of the preceding embodiments, wherein the content of Fe is in the range from about 0.0001 to about 1 wt. %, preferably in the range from about 0.0005 to about 0.1 wt. %, more preferably in the range from about 0.001 to about 0.05 wt. %, based on the total weight of the alloy.

|7| The alloy according to any of the preceding embodiments, wherein at least one of the following is satisfied:

> a) The content of C in the alloy is less than about 0.1 wt. %, preferably less than about 0.08 wt. % more preferably less than about 0.05 wt. %
> b) The content of B in the alloy is less than about 0.01 wt. %, preferably less than about 0.001 wt%, more preferably less than about 0.0002 wt. %;
> c) The content of P in the alloy is less than about 0.01 wt. %, preferably less than about 0.005 wt. %, more preferably less than about 0.001 wt. %, further more preferably less than about 0.0005 wt. %;
> d) The content of S in the alloy is less than about 0.005 wt. %, preferably less than about 0.003 wt. %, more preferably less than about 0.002 wt. %, further more preferably less than about 0.0008 wt. %;

each wt. % being based on the total weight of the alloy. In preferred aspects of this embodiment, the combination of the above criteria which are satisfied is selected from the group consisting of: a), b), c), d), a)+b), a)+c), a)+d), b)+c), b)+d), c)+d), a)+b)+c), a)+b)+d), a)+c)+d), b)+c)+d) and a)+b)+c)+d).

|8| The alloy according to any of the preceding embodiments, wherein at least one of the following is satisfied:

> a) The content of Mn in the alloy is less than about 0.05 wt. %, preferably less than about 0.005 wt. %, more preferably less than about 0.001 wt. %;
> b) The content of Si in the alloy is less than about 0.05 wt. %, preferably less than about 0.03 wt. %, more preferably less than about 0.02 wt. %;

each wt. % being based on the total weight of the alloy. In preferred aspects of this embodiment, the combination of the above criteria which are satisfied is selected from the group consisting of: a), b), a)+b).

|9| The alloy according to any of the preceding embodiments, wherein at least one of the following is satisfied:

> a) The content of O in the alloy is in the range from about 0.0001 to about 0.05 wt. %, preferably in the range from about 0.0001 to about 0.03 wt. %, more preferably in the range from about 0.0001 to about 0.01 wt. %;
> b) The content of N in the alloy is in the range from about 0.0001 to about 0.01 wt. %, preferably in the range from about 0.0001 to about 0.008 wt. %, more preferably in the range from about 0.0001 to about 0.005 wt. %;

each wt. % being based on the total weight of the alloy. In preferred aspects of this embodiment, the combination of the above criteria which are satisfied is selected from the group consisting of: a), b), a)+b).

|10| The alloy according to any of the preceding embodiments, wherein at least one of the following is satisfied:

> a) The alloy contains less than about 0.01 wt. %, preferably less than about 0.005 wt. %, more preferably less than about 0.001 wt. %, O in the form of a magnesium oxide;
> b) The alloy contains less than about 0.01 wt. %, preferably less than about 0.005 wt. %, more preferably less than about 0.001 wt. %, O in the form of an aluminium oxide;
> c) The alloy contains less than about 0.01 wt. %, preferably less than about 0.005 wt. %, more preferably less than about 0.001 wt. %, O in the form of a cerium oxide.
> d) The alloy contains less than about 0.01 wt. %, preferably less than about 0.005 wt. %, more preferably less than about 0.001 wt. %, O in the form of a calcium oxide.
> e) The alloy contains less than about 0.01 wt. %, preferably less than about 0.005 wt. %, more preferably less than about 0.001 wt. %, O in the form of a chromium oxide.
> In preferred aspects of this embodiment, the combination of the above criteria which are satisfied is selected from the group consisting of: a), b), c), d), a)+b), a)+c), a)+d), b)+c), b)+d), c)+d), a)+b)+c), a)+b)+d), a)+c)+d), b)+c)+d), a)+b)+c)+d), e), a)+e), b)+e), c)+e), d)+e), a)+b)+e), a)+c)+e), a)+d)+e), b)+c)+e), b)+d)+e), c)+d)+e), a)+b)+c)+e), a)+b)+d)+e), a)+c)+d)+e), b)+c)+d)+e) and a)+b)+c)+d)+e).

|11| A process for the preparation of an alloy comprising the following preparation steps:

a) Provision of a mixture comprising the following components:

i. Cr in the range from about 10 to about 30 wt. %, preferably in the range from about 15 to about 25 wt. %, more preferably in the range from about 19 to about 21 wt. %;
ii. Ni in the range from about 20 to about 50 wt. %, preferably in the range from about 25 to about 40 wt. %, more preferably in the range from about 33 to about 37 wt. %;
iii. Mo in the range from about 2 to about 20 wt. %, preferably in the range from about 5 to about 15 wt. %, more preferably in the range from about 9 to about 10.5 wt. %;
iv. Co in the range from about 10 to about 50 wt. %, preferably in the range from about 20 to about 40 wt. %, more preferably in the range from about 33 to about 37 wt. %.

wherein each wt. % is based on the total weight of the mixture prepared for melting;
b) Melting the mixture in a vacuum induction melting step in order to obtain a first melt, in one aspect of this embodiment, one or more further vacuum induction melting steps are carried out;
c) Solidifying the first melt in order to obtain a first solid;
d) Melting the first solid in a vacuum arc melting step in order to obtain a further melt;
e) Solidifying the further melt in order to obtain a further solid.

|12| The process according to embodiment |11|, wherein pressure in step b) is below about 0.1 bar, preferably below about 0.05 bar, more preferably below about 0.01 bar.

|13| The process according to embodiment |11| or |12|, wherein the leak rate in step b) is below about 0.1 bar / min, preferably below about 0.05 bar / min, more preferably below about 0.01 bar / min.

|14| The process according to any of the embodiments |11| to |13|, wherein the pressure in step d) is below about 0.05 bar, preferably below about 0.01 bar, more preferably below about 0.005 bar.

|15| The process according to any of the embodiments |11| to |14|, wherein the leak rate in step d) is below about 0.05 bar / min, preferably less than about 0.01 bar / min, more preferably less than about 0.005 bar / min.

|16| The process according to any of the embodiments |11| to |15|, further comprising a homogenisation step carried out at a temperature in the range from about 900 to about 1300 °C, preferably in the range from about 1000 to about 1250 °C, more preferably in the range from about 1100 to about 1225 °C.

|17| The process according to any of the embodiments |11| to |16|, further comprising a cogging step carried out at a temperature in the range from about 900 to about 1300 °C, preferably in the range from about 1000 to about 1250 °C, more preferably in the range from about 1100 to about 1225 °C.

|18| The process according to any of the embodiments |11| to |17|, further comprising a finish roll step carried out at a temperature in the range from about 900 to about 1300 °C, preferably in the range from about 1000 to about 1250 °C, more preferably in the range from about 1100 to about 1225 °C.

|19| The process according to any of the embodiments |11| to |18|, further comprising a straightening step. In one aspect of this embodiment, the straightening is a hot straightening, preferably carried out at a temperature in the range from about 900 to about 1200 °C, preferably in the range from about 950 to about 1100 °C, more preferably in the range from about 1000 to about 1075 °C. In one aspect of this embodiment, the straightening is a cold straightening, preferably carried out at ambient temperature, preferably at a temperature in the range from about 10 to about 100 °C, more preferably in the range from about 15 to about 80 °C, most preferably in the range from about 20 to about 50 °C.

|20| An alloy obtainable by a process according to any of the embodiments |11| to |19|.

|21| An electrical wire comprising an alloy according to any of the embodiments |1| to |10| or |20|.

|22| A medical device comprising a wire according to embodiment |21|.

|23| A pacemaker, an implantable cardioverter defibrillator, a cardiac resyncronisation device, a neuromodulation device, a cochlea implant or any other implantable stimulation device comprising a wire according to embodiment |21|.

Alloy

The Cr, Ni, Mo and Co alloy comprise two or more elements, preferably as a solid mixture, preferably with an enthalpy of mixing of the constituent elements of less than about 10 KJ/mol, preferably less than about 5 KJ/mol, more preferably less than about 1 KJ/mol. The Cr, Ni, Mo and Co alloy comprise Cr, Ni, Mo and Co as major constituents, preferably with at least about 95 wt. %, more preferably at least about 99 wt. %, further more preferably at least about 99.9 wt. %, more preferably at least about 99.95 wt. % of the alloy being Cr, Ni, Mo and Co.

A composition of the Cr, Ni, Mo and Co alloy is preferred which improves favourable properties of the alloy, in particular resistance to fatigue and/or corrosion resistance, preferably both.

It is preferred for the properties of the alloy to be improved by limiting the content of impurities or limiting the content of a combination of different impurities, preferably according the embodiments of the invention.

It is preferred that there be a low, preferably zero concentration of inclusions in the alloy. This is preferably achieved by limiting the content of impurities. In one embodiment it is preferred that the alloy contain less than about 0.01 %, preferably less than about 0.005 %, more preferably less than about 0.001 % inclusions. The % of inclusions is preferably determined using the microscopic inspection method given in the test methods. Content of inclusions as % is there determined as the proportion of the cross sectional area of the sample surface made up of inclusions. In some instances, the alloy comprises a low, preferably a zero concentration of inorganic non-metallic solid inclusions, more preferably of inorganic oxide inclusions. Inorganic oxides in this context can refer to metal oxides, non-metal oxides and metalloid-oxides. In some cases the the alloy comprises a low, preferably a zero concentration of inclusions comprising one or more selected from the group consisting of: Si, Al, Ti, Zr and B; preferably selected form the group consisting of: Si Ti, and Al.

In one embodiment, one or more treating material(s) is/are contacted with the mixture of the process in order to remove oxygen from the mixture of the process, preferably by incorporation of the oxygen into a dross and removal of the dross. Preferred treating materials in this context comprise one or more selected from the list consisting of: Al, Mg, Ca and Ce; preferably in the form of an element and/or in the form of an alloy, wherein the alloy preferably contains a further metal being selected from group consisting of Cr, Ni, Mo and Co or at least two thereof, preferably Ni.

In order to achieve the preferred concentrations of constituents of the alloy, described above in the embodiments, the skilled person may vary the proportions of starting materials employed in the preparation process. The proportions of the starting materials might not be equal to the proportions of constituents of the product, due to net loss or gain during the preparation process.

Process for preparation of the alloy

The process for the preparation of the alloy preferably comprises the following steps:

    a) A vacuum induction melting step;
    b) A vacuum arc melting step.

In one embodiment of the invention, the process comprises two or more vacuum induction melting steps. In another embodiment of the invention, the process comprises two or more vacuum melting steps. In another embodiment of the invention, the process comprises two or more vacuum induction melting steps and two or more vacuum arc melting steps.

In preferred embodiments of the invention, the process further comprises one or more of the following steps:

    c) An electro-slag melting step
    d) A homogenisation step
    e) A cogging step
    f) A finish roll step
    g) A straightening step

In preferred embodiments, the process comprises a combination of the above steps selected from the list consisting of: c), d), e), f), g), c)+d), c)+e), c)+f), c)+g), d)+e), d)+f), d)+g), e)+f), e)+g), f)+g), c)+d)+e), c)+d)+f), c)+d)+g), c)+e)+f), c)+e)+g), c)+f)+g), d)+e)+f), d)+e)+g), d)+f)+g), e)+f)+g), d)+e)+f)+g), c)+e)+f)+g), c)+d)+f)+g), c)+d)+e)+g), c)+d)+e)+f) and c)+d)+e)+f)+g).

In one embodiment of the invention, one or more of the steps c)-g) is carried out two or more times.

In preferred vacuum induction melting steps, a material is heated by inducing an electric current in the material, preferably by electromagnetic induction. The pressure in the vacuum induction melting step is preferably below about 0.1 mbar, more preferably below about 0.01 mbar, most preferably below about 0.001 mbar. The vacuum induction melt step is preferably carried out in an oven, preferably with a low leak rate, preferably below about 0.1 mbar·l/s, more preferably below about 0.01 mbar·l/s, most preferably below about 0.001 mbar·l/s. The leak rate is preferably tested before the vacuum induction melting step by evacuating the oven, closing the valves of the oven, and measuring the rate of increase of pressure in the oven.

In one embodiment of the invention, the vacuum induction melting step is carried out in an inert atmosphere, preferably argon, preferably an atmosphere comprising at least about 90 wt. %, more preferably at least about 99 wt. %, most preferably at least about 99.9 wt. % of inert gas, preferably argon. In one aspect of this embodiment, the oven is evacuated and inert gas, preferably argon, introduced into the oven before melting. In one aspect of this embodiment, the pressure in the vacuum induction melting step is in the range from about 1 to about 200 mbar, preferably in the arrange from about 10 to about 150 mbar, most preferably in the range from about 20 to about 100 mbar.

In preferred vacuum arc melting steps, a material is heated by passing an electrical current through the material, preferably with an electrical power in the range from about 300 to about 1200 W/kg, more preferably in the range from about 400 to about 1000 W/kg, most preferably in the range from about 450 to about 900 W/kg, based on the mass of material heated. The pressure in the vacuum arc melting step is preferably below about 0.1 mbar, more preferably below about 0.01 mbar, most preferably below about 0.001 mbar. The vacuum arc melt step is preferably carried out in an oven, preferably with a low leak rate, preferably below about 0.1 mbar·l/s, more preferably below about 0.05 mbar·l/s, most preferably below about 0.01 mbar·l/s. The leak rate is preferably tested before the vacuum arc melting step by evacuating the oven, closing the valves of the oven, and measuring the rate of increase of pressure in the oven. In one embodiment of the invention, the vacuum arc melting step is carried out in an inert atmosphere, preferably argon, preferably an atmosphere comprising at least about 90 wt. %, more preferably at least about 99 wt. %, most preferably at least about 99.9 wt. % of inert gas, preferably argon. In one aspect of this embodiment, the oven is evacuated and inert gas, preferably argon, introduced into the oven before melting. In one aspect of this embodiment, the pressure in the vacuum arc melting step is in the range from about 0.001 to about 0.2 bar, preferably in the range from about 0.01 to about 0.15 bar, most preferably in the range from about 0.05 to about 0.1 bar.

Homogenisation steps according to the invention preferably allow reduction of inhomogeneity in a material, preferably by heating. In preferred homogenisation steps according to the invention, a material is heated to a temperature which is below its melting temperature, preferably below its incipient melting temperature. It is preferred that the material be homogenised for a duration in the range from about 10 min. to about 20 hours, more preferably in the range from about 3 hours to about 10 hours, most preferably in the range from about 5 hours to about 8 hours. Homogenisation is preferably carried out in a vacuum or in a gaseous atmosphere, preferably in a gaseous atmosphere. It is preferred that the homogenisation step be carried out close to atmospheric pressure, preferably in the range from about 0.5 to about 1.5 bar, more preferably in the range from about 0.8 to about 1.2 bar, most preferably in the range from about 0.9 to about 1.1 bar. In one preferred embodiment, the homogenisation step is carried out in air.

In preferred cogging steps according to the invention, the porosity or grain size or both of a material are reduced, preferably at elevated temperatures, preferably below the melting point of the material, preferably with the application of compressive force. Compressive forces may be applied locally or in a delocalised manner, preferably by one or more selected from the group consisting of: rolling, pressing, beating and turning. Where the material to be cogged has a mass below about 10 kg, preferably below about 8 kg, more preferably below about 5 kg, rolling is preferred. Where the material to be cogged has a mass above about 10 kg, preferably above about 20 kg, more preferably above about 30 kg, beating or turning is preferred. It is preferred that the smallest dimension of the material is reduced during the cogging process.

Preferred finish roll steps according to the invention reduce the smallest dimension of the material, preferably by passing the material through one or more pairs of rolls, preferably below the melting point of the material, more preferably below its incipient melting point. In one embodiment, the finish roll step reduces the porosity or grain size of the material, preferably both.

Straightening preferably reduces the physical curvature of the material, preferably so as to facilitate further grinding or machining steps. Straightening is preferably carried out by applying compressive force. The straightening step is preferably carried out below the melting point of the material, more preferably below its incipient melting point. In one embodiment, the process comprises a hot straightening step. In one embodiment, the process comprises a cold straightening step, preferably carried out at around ambient temperature. Cold straightening is preferably carried out at a temperature in the range from about 10 to about 100 °C, more preferably in the range from about 15 to about 80 °C, most preferably in the range from about 20 to about 50 °C.

Leads, wires and Medical Devices

In this text, reference is made variously to a coated or cladded wire, which comprises a wire core and a shell. The shell might be coated or cladded onto the core wire.

A preferred lead according to the invention comprises at least one proximal connector, at least one distal electrode and a flexible elongated conductor that is electrically connecting the electrode(s) to the connector(s). Preferably the elongated conductor is a coiled wire or a cable and comprises the alloy according to the invention.

A contribution to achieving at least one of the above mentioned objects is made by a wire comprising an alloy according to the invention, preferably having a thickness in the range from about 10 to about 50 $\mu$m, preferably in the range from about 15 to about 35 $\mu$m. In one embodiment, the wire further comprises silver metal.

In one embodiment, the lead comprises a silver core and an alloy according to the invention, preferably present as a shell surrounding the silver core.

A contribution to achieving at least one of the above mentioned objects is made by a lead comprising one or more wires according to the invention, preferably grouped into two or more cables, each cable comprising two or more wires according to the invention. In one embodiment, the cables have a thickness in the range from about 0.05 to about 0.5 mm, preferably in the range from about 0.1 to 0.4 mm.

A contribution to achieving at least one of the above mentioned problems is made by a medical device, preferably a pacemaker, comprising a lead according to the invention. A preferred pacemaker comprises:

- An implantable pulse generator;

- One or more leads according to the invention.

In one embodiment, the pacemaker comprises one or more pulsers.

In one embodiment, the pacemaker comprises one or more energy cells, preferably one or more electrical cells.

A process for the preparation of a wire comprises the steps:

a) Providing a tube of alloy according to the invention;
b) At least partially filling the tube with Ag to obtain a composite;
c) One or more drawing steps to reduce the diameter of the composite;
d) Optionally one or more annealing steps to soften the composite and facilitate drawing.

In one embodiment of the invention, the Ag content of the wire obtainable by the process is in the range from about 15 to about 50 wt. %, preferably in the range from about 17.5 to about 45.7 wt. %, more preferably in the range from about 28.7 to about 37.7 wt. %, based on the total weight of the wire.

In one embodiment, the diameter of the wire obtainable by the process is in the range from about 5 to about 50 $\mu$m, preferably in the range from about 15 to about 35 $\mu$m.

In one embodiment, the filling degree of silver in the wire obtainable by the process is in the range from about 15 % to about 41 %, preferably in the range from about 20 % to about 35 %, more preferably in the range from about 23 % to about 33 %.

It shall be understood that the method for manufacturing a cable, the cable manufactured by such method, and the medical device comprising such cable according to the independent claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

Description of the Drawings

The invention is now further illustrated using figures which are not to be considered as limiting the scope of the invention.

Figure 1 shows schematically a lead according to the invention.
Figure 2 shows schematically an apparatus for measuring fatigue resistance.
Figure 3 shows schematically a pacemaker comprising a lead according to the invention.
Figure 4 shows a cross sectional image of a wire of material according to example 2 (comparative).
Figure 5 shows a cross sectional image of a wire of material according to example 2 (comparative).
Figure 6 shows an analysis of elemental composition by energy dispersive x-ray spectroscopy of an inclusion in a wire of material according to example 2 (comparative).

Figure 7 shows a cross sectional image of a wire of material according to example 2 (comparative).

Figure 8 shows a cross sectional image of a wire of material according to example 2 (comparative).

Figure 9 shows a cross sectional image of a wire of material according to example 2a (comparative) with an Ag core.

Figure 10 shows a cross sectional image of a wire of material according to example 2a (comparative) with an Ag core.

Figure 11 shows an analysis of elemental composition by energy dispersive x-ray spectroscopy of an inclusion in a wire of material according to example 2a (comparative) with an Ag core.

Figure 12 shows a plot of fatigue results for a wire of material according to example 1 (inventive) and a wire of material according to example 2 (comparative).

Figure 13 shows a plot of fatigue results for a wire of material according to example 1a (inventive) with an Ag core and a wire of material according to example 2a (comparative) with an Ag core.

Figure 14 shows a method for manufacturing a cable.

Figure 15 shows different configurations of a cable.

Figure 16 shows a LCF fatigue comparison. Figure 17 shows a HCF fatigue comparison.

Figure 18 shows a LCF comparison.

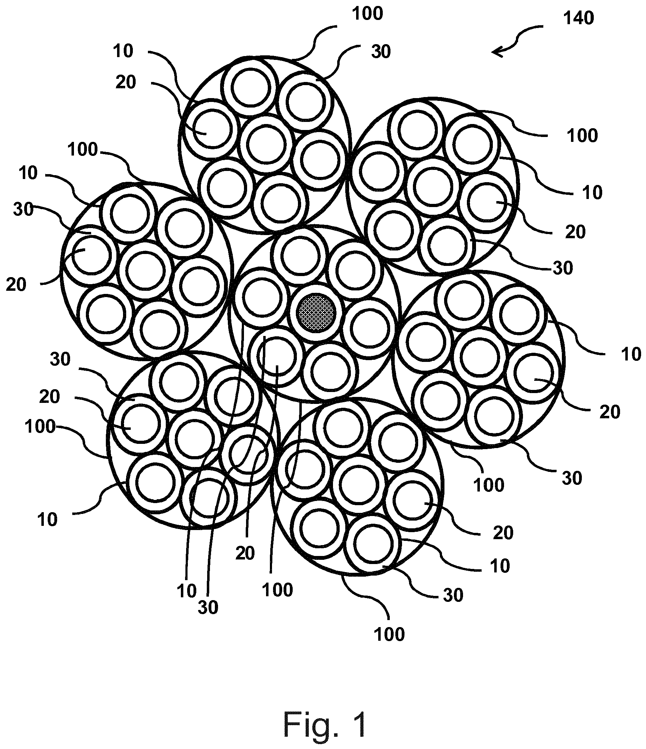

Figure 1 shows schematically a lead having a cable bundle 140, which comprises cables 100. In this example, the cables 100 each comprise 7 wires 10. Each wire comprises a first region 20 and a further region 30, wherein the first region 20 is interior to the region 30 along the length of the lead 140. The first region 20 is 41 area % of the cross sectional area the wire 10 and the further region is 59 area % of the cross sectional area of the wire 10, in each case based on the total cross sectional area of the wire 10. In this example, the first region 20 is silver. The further region 30 is a Cr, Ni, Mo and Co alloy as described above. In this example, the cable bundle 140 comprises 7 cables 100, each cable 100 comprising 7 wires 10. The invention is not limited to this arrangement. In particular, other arrangements of wires 10 in cables 100 and/or other arrangements of cable bundles 140 in leads are conceivable.

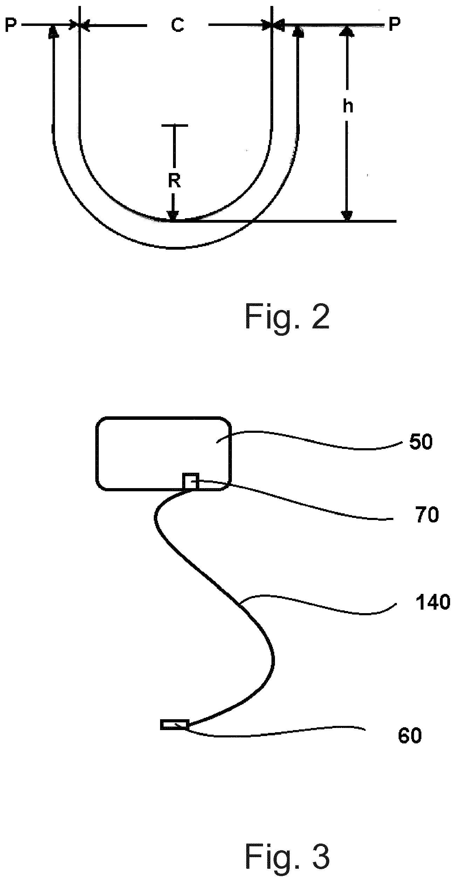

Figure 2 shows schematically an apparatus for measuring fatigue resistance.

Figure 3 shows schematically a pacemaker 50 with a pulse generator 70, and a lead 140 comprising an electrode 60. The lead 140 connects the pulse generator 70 and the heart tissue via the electrode 60.

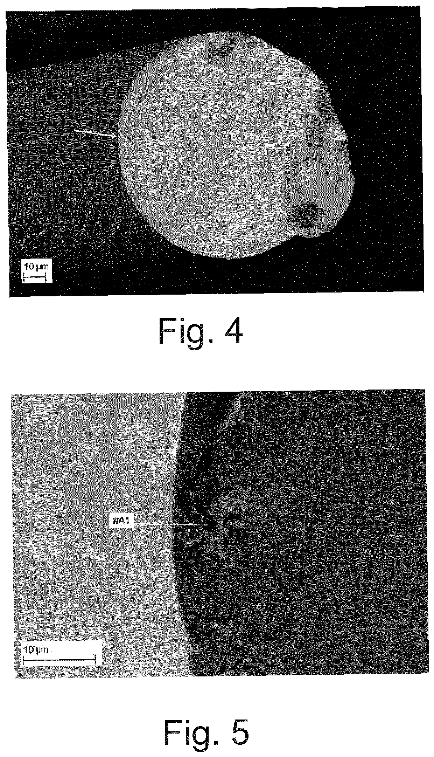

Figure 4 shows a cross sectional image of a wire of material according to example 2 (comparative) as observed by backscattered electron imaging according to the test method. A dark inclusion is indicated with an arrow.

Figure 5 shows a cross sectional image of a wire of material according to example 2 (comparative) as observed by backscattered electron imaging according to the test method. Figure 5 shows the same image as figure 4, but at higher magnification. A dark inclusion is indicated with the reference mark #A1.

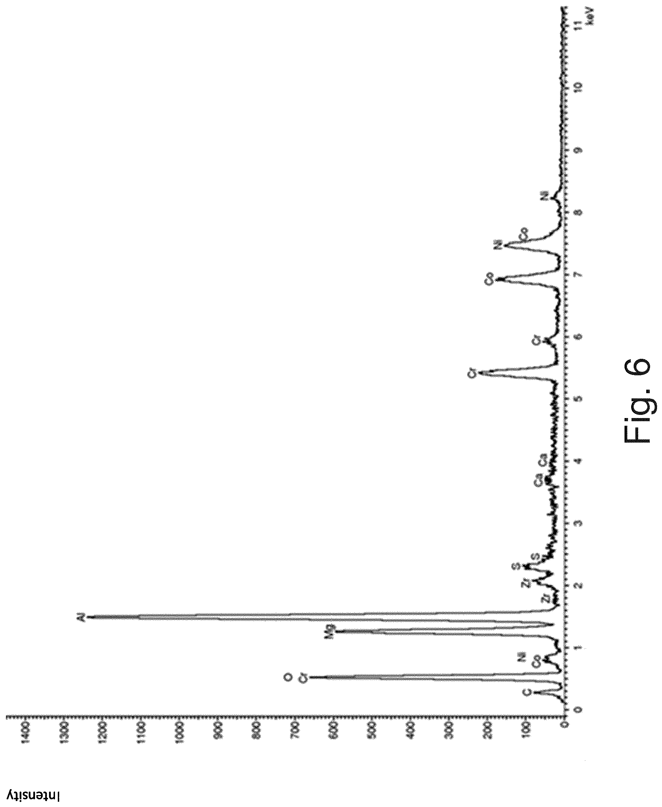

Figure 6 shows an analysis of elemental composition by energy dispersive x-ray spectroscopy according to the fracture surface analysis test method of the surface of an inclusion in a wire of material according to example 2 (comparative). The surface analysed is the inclusion indicated as #A1 in figure 5. In particular, the analysis shows the presence of Al and Mg impurities and also of entities with a Cr-O bond.

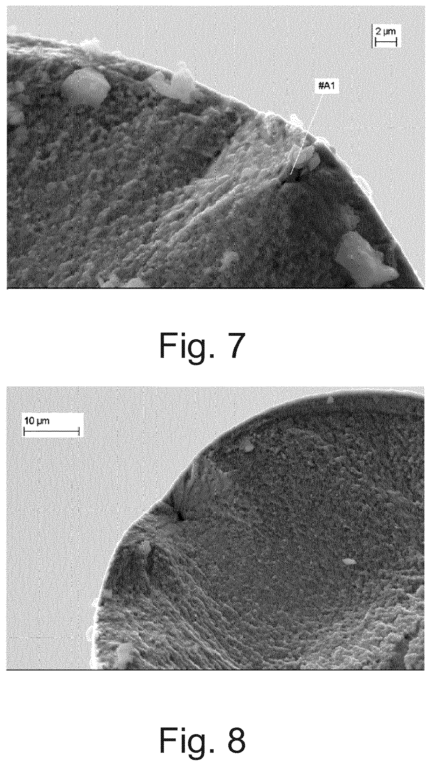

Figure 7 shows a cross sectional image of a wire of material according to example 2 (comparative) as observed by backscattered electron imaging according to the test method. A dark inclusion is indicated with the reference mark #A1.

Figure 8 shows a cross sectional image of a wire of material according to example 2 (comparative) as observed by backscattered electron imaging according to the test method. The surface shown in figure 8 is taken from the same slice as that of figure 7.

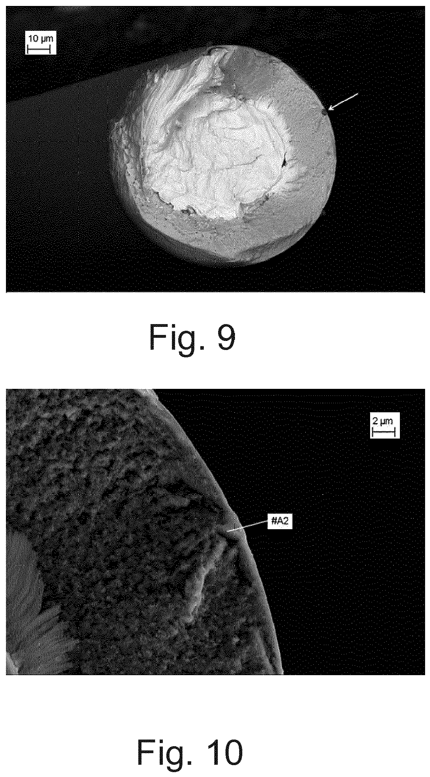

Figure 9 shows a cross sectional image of a wire of material according to example 2a (comparative) with an Ag core, as observed by backscattered electron imaging according to the test method. A dark inclusion is indicated with an arrow.

Figure 10 shows a cross sectional image of a wire of material according to example 2a (comparative) with an Ag core, as observed by backscattered electron imaging according to the test method. Figure 10 shows the same image as figure 9, but at higher magnification. A dark inclusion is indicated with the reference mark #A2.

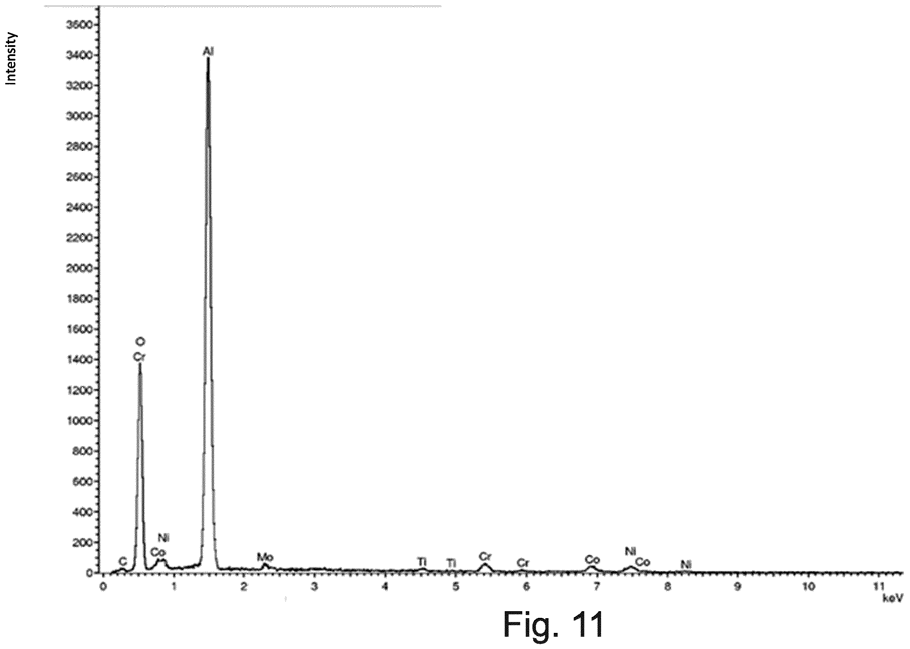

Figure 11 shows an analysis of elemental composition by energy dispersive x-ray spectroscopy according to the fracture surface analysis test method of the surface of an inclusion in a wire of material according to example 2a (comparative) with an Ag core. The surface analysed is the inclusion indicated as #A2 in figure 10. In particular, the analysis shows the presence of Al impurities and also of entities with a Cr-O bond.

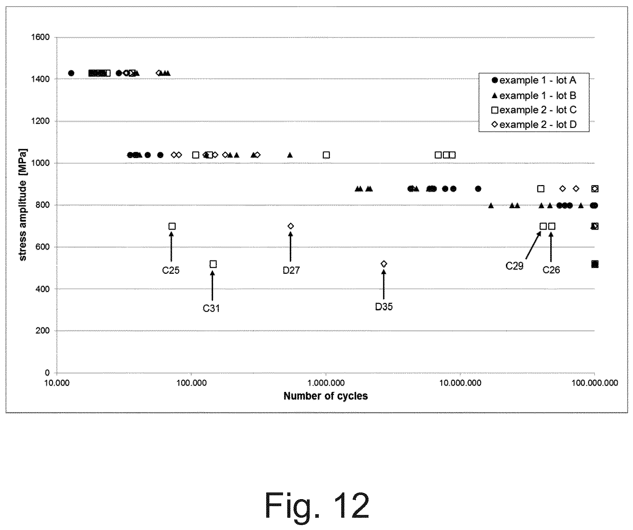

Figure 12 shows a plot of fatigue results for a wire of material according to example 1 (inventive) and a wire of material according to example 2 (comparative). For example 1 (inventive), results are shown for 2 lots, lot A as represented by a solid circle and lot B as represented by a solid triangle. For example 2 (comparative), results are shown for 2 lots, lot C as represented by a hollow square and lot D as represented by a hollow diamond. The number of cycles before failure is shown as dependent on the stress amplitude applied in the test. Outliers, which performed poorly are indicated with arrows.

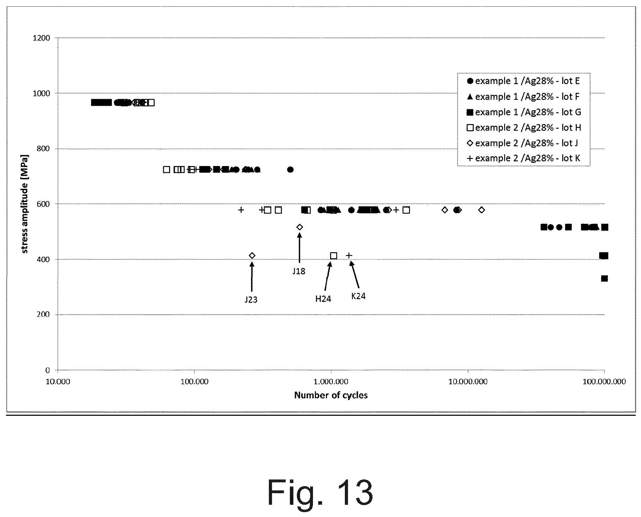

Figure 13 shows a plot of fatigue results for a wire of material according to example 1a (inventive) with an Ag core and a wire of material according to example 2a (comparative) with an Ag core. For example 1a (inventive), results are shown for 3 lots, lot E as represented by a solid circle, lot F as represented by a solid triangle and lot G as represented by a solid square. For example 2a (comparative), results are shown for 3 lots, lot H as represented by a hollow square, lot J as represented by a hollow diamond and lot K as represented by a cross. The number of cycles before failure is shown as dependent on the stress amplitude applied in the test. Outliers, which performed poorly are indicated with arrows.

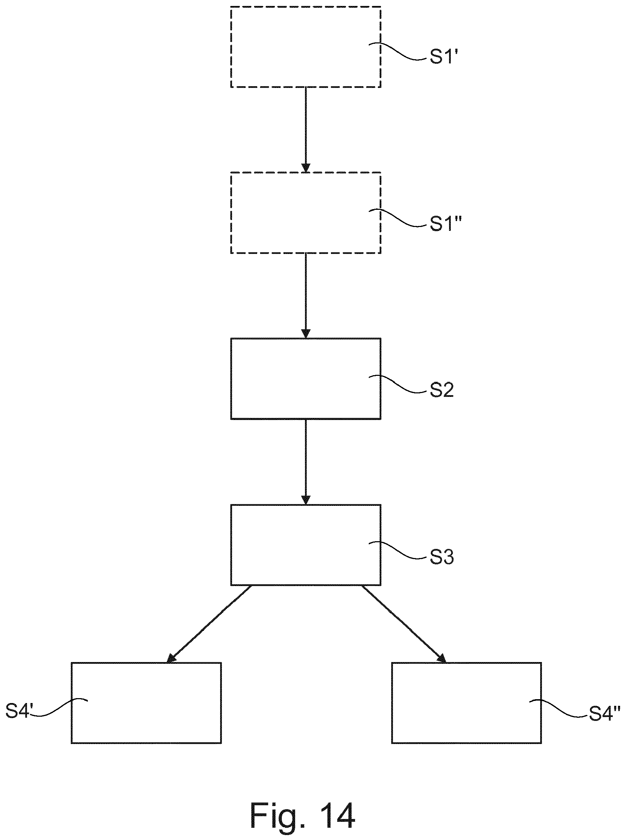

Figure 14 shows a method for manufacturing a cable 100. The method for manufacturing the cable 100 comprises the following steps:

In step S1,    providing several raw wires made of a wire material,
In step S2,    drawing the raw wires into wires 10,
In step S3,    coiling the wires 10 into a cable 100, and
In step S4,    heat treating the cable 100.

Step S1 may be a simple provision of raw wires, but may also be an optional step of an initial drawing with e.g. a cold working percentage of 95 to 96 % as step S1' and of an initial heat treatment as step S1" with e.g. 875 to 950 °C and a dwell time of 5 to 8s or 950 to 1100 °C and a dwell time of 7 to 15s.

Step S2 is here a drawing with a cold working percentage of 98 to 99 % to e.g. a wire outer diameter of 25 to 27 μm.

Step S3 is here a coiling to e.g. a 1x7 configuration with e.g. a wire outer diameter of 76 to 78 μm, a 7x7 configuration with e.g. a wire outer diameter of 270 to 275 μm, and a 1x19 configuration with e.g. a wire outer diameter of 125 to 128 μm.

Step S4 may be a heat treating step S4' at e.g. 480 to 580°C for improving low cycle fatigue or a step S4" at e.g. 780 to 810° for improving high cycle fatigue and ductility.

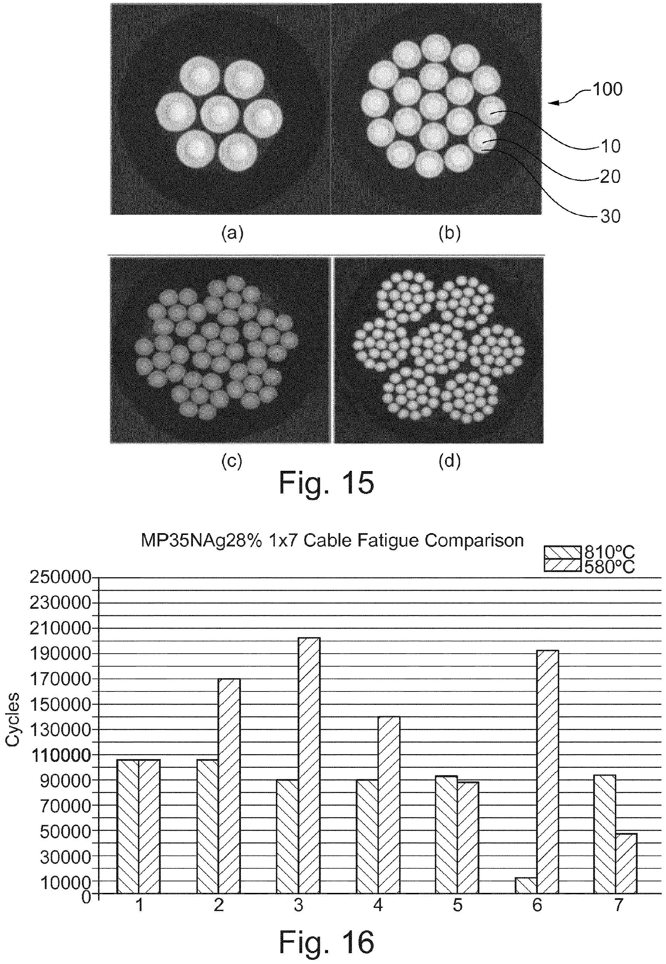

Figure 15 shows different configurations of a cable 100, namely (a) a 1x7 cable, (b) a 1x19 cable, (c) a 7x7 cable and (d) a 7x19 cable. All cables comprise several wires 10 and each wire is here a composite wire out of an additional metallic material as e.g. Silver as a first region 20 or core and the Cr, Ni, Mo and Co alloy as a further region 30 or shell when the wire is seen in a cross section.

Cables 100 were subjected to a 90° reverse bend flex fatigue tester for a varying stress amplitudes by changing a diameter of mandrels, with the lower diameter mandrel used for applying high stress on the cables and vice versa. The cables were bend across the mandrel to the specified radius by adjusting the distance between the mandrels and an electrical signal is connected between the two ends of the cable. Once the cable breaks, the electrical signal is terminated between the two ends of the cable and the respective counter counting the number of cycles stops.

As shown in the following table, a higher YS/UTS ratio and a higher ductility are found in a 1x7 cable configuration out of MP35N/Ag-28% composite wires (Cr, Ni, Mo and Co alloy as shell with a Silver core with a filling ratio of 28 %) when annealed at a higher temperatures with a CW of 99% and stress relieved at various temperatures for a dwell time of 5 to 6 seconds.

Mechanical properties comparison of a 1x7 cable configured with 25 μm drawn wires with a CW of 99% to make a cable outer diameter of 76 μm and subjected to several stress relief temperatures

| Temp | YS(MPa) | YS(Ksi) | UTS(Mpa) | UTS(Ksi) | EL | YS/UTS |
|------|---------|---------|----------|----------|-----|--------|
| Bare Cable | 1852 | 268.6101744 | 1904 | 276.1521447 | 1.87 | 1.028078 |
| 350 | 1692 | 245.4041118 | 1826 | 264.8391892 | 1.91 | 1.079196 |
| 450 | 1781 | 258.3124841 | 1934 | 280.5032815 | 1.96 | 1.085907 |
| 500 | 1842 | 267.1597955 | 1977 | 286.7399108 | 1.95 | 1.07329 |
| 580 | 2009 | 291.3811233 | 2033 | 294.8620327 | 1.92 | 1.011946 |
| 650 | 2027 | 293.9918054 | 2043 | 296.3124116 | 1.89 | 1.007893 |

(continued)

| Temp | YS(MPa) | YS(Ksi) | UTS(Mpa) | UTS(Ksi) | EL | YS/UTS |
|---|---|---|---|---|---|---|
| 700 | 1949 | 282.6788498 | 2013 | 291.9612749 | 1.95 | 1.032837 |
| 750 | 1707 | 247.5796802 | 1851 | 268.4651365 | 2.12 | 1.084359 |
| 810 | 1190 | 172.5950905 | 1405 | 203.7782371 | 2.93 | 1.180672 |

The same configuration cable as above, but the wires used to make a 1x7 strand were subjected to a CW% of 80 to 86 % lead to a lower YS/UTS ratio and a higher drop in ductility when stress relieved to different temperatures for a dwell time of 5 to 6 seconds as shown in the table below when compared to the table above.

Mechanical properties comparison of the 1x7 cable configured with 25 $\mu$m drawn wires with a CW of 86 % to make a cable outer diameter of 76 $\mu$m and subjected to several tress relief temperatures

| Temp | YS(MPa) | YS(Ksi) | UTS(Mpa) | UTS(Ksi) | EL | YS/UTS |
|---|---|---|---|---|---|---|
| Bare cable | 1364 | 197.832 | 1652 | 239.603 | 2.13 | 0.82567 |
| 300 | 1534 | 222.488 | 1660 | 240.763 | 1.82 | 0.9241 |
| 350 | 1596 | 231.48 | 1694 | 245.694 | 1.78 | 0.94215 |
| 450 | 1705 | 247.29 | 1734 | 251.496 | 1.71 | 0.98328 |
| 500 | 1750 | 253.816 | 1762 | 255.557 | 1.72 | 0.99319 |
| 580 | 1824 | 264.549 | 1880 | 272.671 | 1.72 | 0.97021 |
| 650 | 1810 | 262.519 | 1911 | 277.167 | 1.65 | 0.94715 |
| 750 | 1919 | 278.328 | 1946 | 282.244 | 1.78 | 0.98613 |
| 800 | 1851 | 268.465 | 1918 | 278.183 | 1.78 | 0.96507 |
| 825 | 1622 | 235.251 | 1765 | 255.992 | 2.34 | 0.91898 |

Figure 16 shows a LCF fatigue comparison of MP35N/Ag-28% alloy 1x7 cables annealed at different stress relief temperatures and subjected to a 90° reverse bend fatigue tests on 1.6 mm diameter mandrels. Higher LCF fatigue performance on the 1x7 cables was observed when stress was relieved at a temperature of 580°C when compared to cables annealed at higher temperature of 810°C as shown in Figure 16 when subjected to a reverse bend fatigue test on 1.6 mm diameter mandrels. This can be attributed to the thermo-mechanical processing characteristics of the cable, which might have led to an arrest of crack propagation due to its small grain structure and the concentration of dislocations and twins at the grain boundaries, which might have arrested the crack propagation.

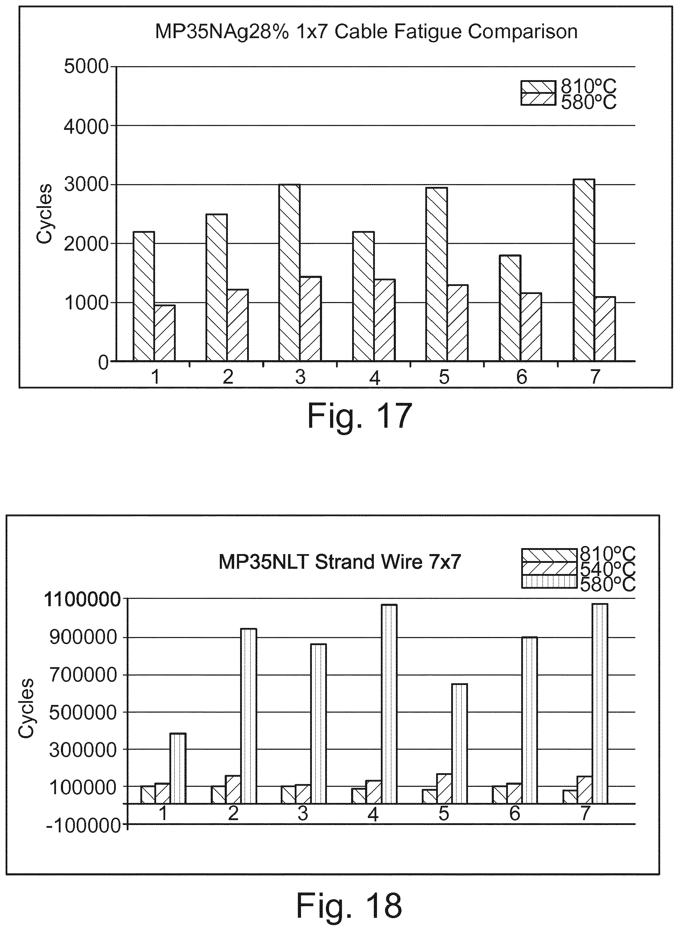

Figure 17 shows a HCF fatigue comparison of MP35N /Ag-28% alloy 1x7 cables annealed at different stress relief temperatures and subjected to 90° reverse bend fatigue tests on 1.0 mm diameter mandrels. Higher HCF fatigue performance on the 1x7 cables was observed when stress was relieved at a temperature of 810°C when compared to the cables annealed at temperature of 580°C as shown in Figure 17 when subjected to a reverse bend fatigue test on 1.0 mm diameter mandrels. This can be attributed to the thermo-mechanical processing characteristics of the cable, which might have led to an arrest of crack initiation due to its coarse grain structure at the surface of the cables when annealed at higher temperatures thus leading to a higher HCF performance when compared to cables annealed at lower temperatures of 580°C, where crack initiation is faster due to its small grain sizes and a presence of severe low angle grain boundaries.

Figure 18 shows a LCF fatigue comparison of MP35NLT alloy 7x7 cables annealed at different stress relief temperatures and subjected to 90° reverse bend fatigue tests on 1.6 mm diameter mandrels. As can be seen in Figure 18, higher stress relief temperature annealing lowered a fatigue behavior of the strands drastically.

Test Methods

Alloy composition

For a quantitative chemical analysis of the alloy, the following methods are used:

a) the main components of the alloy (Co, Cr, Ni, Mo) are measured by X-ray fluorescence XRF using the XRF Lab Report - S8 TIGER from the company BRUKER (Bruker AXS GmbH Ostliche Rheinbrückenstr. 49, 76187 Karlsruhe, Germany)
b) Trace elements present in the alloy (Mn, P, Si, Fe, Ti, Al, B, Mg, Ca, Ce, Ti) are measured by glow discharge mass spectrometry (GDMS) using the ASTRUM from Nu Instruments (Nu Instruments Limited, Unit 74, Clywedog Road South, Wrexham, LL13 9XS UK.)
c) Gas or non-metallic components in the alloy (H, O, C, N, S) are measured by carrier-gas hot extraction using the ONH836 from LECO (LECO Corporation, 3000 Lakeview Avenue, St. Joseph, Michigan 49085)

Leak rate

The leak rate of the furnace chamber is measured using the following procedure:

The Vacuum furnace chamber is evacuated to the required pressure by a vacuum pumping station. When the required pressure is reached, the pressure valve between the vacuum furnace chamber and the vacuum pumping station is closed. The pressure increase of the vacuum furnace chamber over a given length of time defines the leak rate of the equipment.

Fatigue resistance

Rotating beam fatigue testing was carried out using Valley Instruments model # 100 test machine (Figure 2) according to Valley Instruments Wire Fatigue Tester Model # 100 user manual (Valley Instruments (Division of Positool Technologies, Inc.), Brunswick, Ohio, USA. Fatigue Tester Model 100 Manual). The equipment consists of a synchronous motor rotating at 3600 rpm. For each test of a wire specimen, a sample having a predefined length is fixed in a custom fine-wire collet at one end, looped through a complete 180 degree turn and is placed at the other end in a low-friction bushing in which it is free to rotate. The synchronous motor of the test device is directly clocked by a counter where the number of cycles is shown in a LCD-display. The fatigue testers are equipped with a sensor to detect the wire fracture which automatically stops the timer, means the display of the timer shows the number of cycles until failure. If no fracture occurs within 100 Million cycles, the test is stopped.

Valley Instruments Wire Fatigue Tester Model # 100 user manual (Valley Instruments (Division of Positool Technologies, Inc.), Brunswick, Ohio, USA. Fatigue Tester Model 100 Manual) describes that a loop, formed by an elastic length held so that the axes of the specimen at the point of retention are exactly parallel, assumes a shape in which:

(1) The length of the loop is 2.19 times the base,
(2) The height of the arch is always 0.835 times the base,
(3) The minimum radius of the curvature occurs at the apex of the arch and is exactly 0.417 times the base, and
(4) The bending stress at the point of minimum curvature bears a simple reciprocal linear relationship to any of the four physical dimensions (length, height, base, and minimum curvature).

The following formulas express the exact relationship:

$$C = 1.198 * E * d / S$$

$$h = 0.835 * C$$

$$L = 2.19 * C$$

$$R = 0.417 * C$$

$$P = 0.141 * E * d^4 / C^2$$

Nomenclature:

C = chuck to bushing distance
d = diameter of wire
h = height of loop
E = modulus of elasticity
L = length of wire external to chucks
R = minimum of radius of curvature
S = bending stress
P = bushing load or lateral force at the chuck

With the above listed formula, the bending stress S (at the peak of the loop) can be calculated by the following equation:

$$S = 1.198 * E * d / C$$

The machine set-up involves calculating the desired sample length and center distance using the modulus of elasticity of the material and equations developed by Valley Instruments Company (user manual).

Microscopic Inspection Method for Micro-cleanliness

Definition: Inclusions are defined as internal flaws or contaminations (such as nitrides or oxides) within the billet or rod from which the wire or tube is produced. The transverse inclusion size is defined as the largest dimension of an internal flaw measured on transverse cross-sections of the billet, rod or wire. The longitudinal inclusion size is defined as the largest dimension of an internal flaw measured on longitudinal cross-sections of the billet, rod or wire. A cross-section diametral line is defined as any line within the cross-section having a length equal to or greater than 95% of the true cross-section diameter.

General Test procedure:

a) Sectioning

For each material lot, the billet, rod or wire is to be sectioned at each end so that there are an equal number of cross sections sampled at the one end as there are samples at the other end (number of samples taken from each end shall differ by no more than one). The total number of cross sections samples depends on the diameter of the billet, rod or wire and is specified in Table 1. The length of each cross section is to be less than its diameter.

b) Imaging

For each billet, rod or solid wire cross-section, non-overlapping images are to be taken at 500X magnification along diametral lines so that the total examined area per sample is at least 1.77 mm$^2$. A cross-section diametral line is defined as any line within the cross-section having a length equal to or greater than 95% of the true cross-section diameter. Angular separation between two diametral lines on a cross- section shall be a minimum of 60 degrees. The number of images and the number of diametral lines depends on the diameter of the billet, rod or wire and is specified in Table 1.

The total number of images is shown in Table 1 and was calculated based on the number of images per sample and the number of samples.

c) Measurement

Each of the images is to be inspected to detect the presence of inclusions or strings of inclusions that exceed a size of 3.0 $\mu$m in their largest dimension. The image inspection may be accomplished either by manual examination or by automated scanning.

**Table 1**

| cross section diameter of billet, rod or wire | | Number of diametral lines per section (no requirement for tube samples) | Number of images per section | Number of cross-sections | | Total images per lot | |
|---|---|---|---|---|---|---|---|
| equal to or greater than [mm] | but no greater than [mm] | | | transverse | longitudinal | transverse | longitudinal |
| 2.54 | 3.80 | 5 | 40 | 12 | 12 | 480 | 480 |
| 3.81 | 5.71 | 3 | 40 | 12 | 12 | 480 | 480 |
| 5.72 | 11.42 | 2 | 40 | 12 | 12 | 480 | 480 |
| 11.43 | 13.96 | 1 | 40 | 12 | 12 | 480 | 480 |
| 13.97 | 17.14 | 1 | 48 | 10 | 10 | 480 | 480 |
| 17.15 | 21.58 | 1 | 60 | 8 | 8 | 480 | 480 |
| 21.59 | 27.93 | 1 | 80 | 6 | 6 | 480 | 480 |
| 27.94 | 33.01 | 1 | 96 | 5 | 5 | 480 | 480 |
| 33.02 | 43.17 | 1 | 120 | 4 | 4 | 480 | 480 |
| 43.18 | 57.14 | 1 | 160 | 3 | 3 | 480 | 480 |

Fracture surface analysis of wire samples

The test method to analyse fracture surfaces of fatigue tested samples was Scanning electron microscopy (SEM). A Zeiss Ultra 55 Gemini was used for the sample analysis of the present invention and comparative samples. Two imaging modes were used to analyse and illustrate the tested samples.

a) SE: the detection of secondary electrons (SE) results in images with a well-defined, three-dimensional appearance. The surface topography can be illustrated in high resolution. Figures 5, 7, 8 and 10 are secondary electron images.

b) BSE: backscatter electrons (BSE) are used to detect contrast between areas with different chemical compositions. Heavy elements (high atomic number) backscatter electrons more strongly than light elements (low atomic number), and thus appear brighter in the image. Figures 4 and 9 are BSE images.

Energy-dispersive X-ray spectroscopy (EDS, EDX) was used for the elemental analysis of features (inclusions/particles) found on the fatigue resistance test samples. A high-energy beam of electrons is focused onto the location of the sample being analysed. This leads to the emission of characteristic X-rays which allows the elemental composition of the feature (inclusions/particles) to be measured. Figures 6 and 11 show EDX scans.

Examples

The MP35N heats were VIM-VAR melted, to minimize the impurity content and to obtain a sound ingot with good chemical uniformity and metallurgical properties. The chemistry of representative heats: Heat 1, Heat 2 and Heat 3 are listed in Table 3. The table also provides the chemistry of a VIM-VAR melted, commercially available MP35N alloy and for reference the chemical requirements per ASTM F562-13, a standard specification for wrought MP35N alloy. The major constituents of MP35N alloy are Co, Ni, Cr and Mo. The new alloy heats were melted in 2 steps. The first melting step was Vacuum Induction Melting (VIM). The VIM furnace consists of a water cooled vacuum melt chamber, an oxide ceramic crucible held in a cylindrical induction heating coil inside the melt chamber, an AC electric power supply, a vacuum pumping system, a raw material adding chamber and a cylindrical metal mold held below and offset from the crucible-induction coil assembly. The vacuum melt chamber, raw material adding chamber and vacuum pumping system are separated by isolation valves. The induction heating coil is water cooled. Electric current from the power supply passes through the induction heating coil creating a magnetic field inside the furnace. The magnetic field induces eddy currents inside the raw materials causing Joule heating. Joule heating raises the temperature of the raw materials to above their melting point. The magnetic field mixes the liquid raw materials to make a homogeneous alloy. The crucible is tilted to pour the liquid alloy from the crucible into the mold. The alloy cools to a solid in the mold under vacuum and is removed from the furnace. The alloy ingot is removed from the mold and it is prepared for re-melting.

For the example heats, 136 kilograms of elemental raw materials were placed in the furnace in proportions calculated to make the aim chemistry. The VIM furnace was closed and pumped down to ≤ 0.00001 bar. A leak-up rate was measured after reaching the desired vacuum pressure level to ensure a vacuum tight furnace. The leak-up rate was ≤ 0.00001 bar/min. Electric power was applied to the induction heating coil. Once the melt was in progress, the vacuum level was recorded at specified intervals to monitor the progress of melting and the mixing and reaction of all of the raw materials. When the reactions ceased as indicated by a constant vacuum pressure level, the heat was poured into a 152.4 mm diameter cylindrical mold.

Each heat was subsequently re-melted by a Vacuum Arc Re-melting (VAR) process to make an 203.2 mm diameter ingot. The VAR furnace consists of water cooled vacuum chamber, a 203.2 mm diameter water cooled copper crucible, a direct current electric power supply, a vacuum pumping system, isolation valves and a computer based electrical system to monitor and control the application of current to the electrode inside the vacuum chamber. The furnace was pumped down to ≤ 0.000006 bar before carrying out the leak-up rate test. A leak rate of ≤ 0.000006 bar /min was obtained. The electrode was moved to a close proximity to the bottom of the crucible. Electric power was applied at a level to cause an electric arc to be struck between the crucible bottom and the alloy electrode. The electric arc causes the electrode to melt and drip into the bottom of the crucible creating a liquid metal pool that solidifies as the arc moves away from the molten pool. The process was continued at a controlled rate until the electrode was consumed. The power was turned off and the ingot was cooled under vacuum. The ingot was removed from the furnace for processing to product.

The as-cast ingot was charged into a gas-fired front opening box furnace with ambient air atmosphere. The furnace was preset to a temperature of 815°C. Upon equilibration of furnace temperature, the ingot was held for additional 4 hours prior to raising the furnace temperature.

The ingot was then heated to 1177°C at a heating rate of 200 K per hour. The ingot was held for 7 hours at 1177°C for homogenization. After homogenization, the ingot was hot rolled from 203 mm to 137 mm round cornered square (RCS) billet using a 559 mm diameter Morgenshammer Mill operating at ambient temperature. The Morgenshammer Mill is a manually operated tilt table mill with 3 high rolls allowing heavy bar to be rolled alternately between the bottom and middle roll and the top and middle roll. After hot rolling the RCS billet was air cooled, abrasively ground by hand to remove surface imperfections and cut to square the ends.

The billet was reheated and hot rolled to 51 mm RCS at 1177°C on the 559 mm Morgenshammer Mill. The RCS was cut to shorter lengths of final rolling on a hand operated 406 mm diameter Morgenshammer Mill with 3 high rolls. All bar manipulation on this mill is done by hand at floor level. The RCS was reheated at 1177°C and rolled to 33.4 mm round bars and air cooled to ambient temperature. The rolled bars were then reheated to 1038°C and held for 30 minutes for hot rotary straightening. After straightening, the bars were air cooled to room temperature. The bars were rough centerless ground, ultrasonic tested for voids and then centerless ground to final size.

For manufacturing of clad-wires, the grinded bars were gun-drilled to produce hollows for subsequent tube drawing. Tubes were filled with Ag-rods and cold-drawn using diamond dies and mineral oil. For a final wire diameter of 127 $\mu$m, the last intermediate annealing was carried out at a wire diameter of 157.5 $\mu$m at 900 - 950 °C in Argon atmosphere. From the last intermediate annealing until the final diameter of the wire, 35% cold-work were applied. Three wire lots were manufactured having UTS values of 1456, 1469 and 1474 MPa. For bare wire, the bars were further hot-rolled to 0.2 inch outer diameter followed by cold-drawing. For 102 $\mu$m final size wire, the last intermediate annealing was carried out at a wire diameter of 122 $\mu$m at 1100 °C in Argon atmosphere to apply 30% cold-work to the final size. Two wire lots were manufactured having UTS values of 1870 and 1875 MPa. The wires of inventive example 1 (Lots A & B) and the cladded wires of inventive example 1a (Lots E, F & G) were made using the alloy of Heat 1 in table 3. The wires of comparative example 2 (lots C & D) and the cladded wires of comparative example 2a (lots H, J & K) were made from the alloy of the commercial heat in table 3 obtained from Fort Wayne Metals, Inc., USA under the trade name 35 NLT®.

**Table 2**

| Material | Example 1 (inventive) | | Example 1a with 28 % Ag (inventive) | | |
|---|---|---|---|---|---|
| Batch | Lot A | Lot B | Lot E | Lot F | Lot G |
| UTS [MPa] | 1870 | 1875 | 1456 | 1469 | 1474 |
| YM [GPa] | 190 | 191 | 121 | 121 | 122 |
| Elongation [%] | 2.8 | 2.9 | 2.2 | 2.3 | 2.3 |

The processed alloy was also obtainable from SAES Smart Materials, Inc. Alloys for the further examples were acquired from SAES Smart Materials, Inc.

**Table 3**

| Element | Heat 1 | Heat 2 | Heat 3 | Commercial Heat | ASTM F-562-13 |
|---|---|---|---|---|---|
| | Wt. % | Wt. % | Wt. % | Wt. % | Wt. % |
| C | 0.0039 | 0.0091 | 0.0106 | 0.005 | < 0.0250 |
| B | 0.000065 | 0.000067 | 0.000008 | 0.01 | < 0.015 |
| P | 0.00018 | 0.000095 | 0.000056 | 0.001 | < 0.015 |
| S | 0.00056 | 0.00026 | 0.00036 | 0.001 | < 0.010 |
| Mn | 0.00028 | 0.00021 | 0.00013 | 0.017 | < 0.15 |
| Si | 0.0042 | 0.0053 | 0.0061 | 0.034 | < 0.15 |
| Al | 0.00023 | 0.00054 | 0.00043 | 0.023 | NA |
| Mg | <0.000001 | 0.000003 | 0.000005 | 0.001 | NA |
| Ca | <0.000005 | <0.000005 | <0.000005 | NA | NA |
| Ce | <0.000001 | <0.000001 | <0.000001 | NA | NA |
| Fe | 0.021 | 0.023 | 0.023 | 0.08 | < 1 |
| Ti | 0.00017 | 0.000038 | 0.000023 | 0.001 | < 1 |
| O | 0.0085 | 0.0056 | 0.0035 | 0.0021 | NA |
| N | 0.0022 | 0.0009 | 0.0007 | 0.0022 | NA |
| Cr | 19.6 | 19.7 | 20 | 20.62 | 19 - 21 |
| Ni | 35.7 | 34.8 | 34.9 | 34.91 | 33 - 37 |
| Mo | 10 | 9.93 | 9.7 | 9.47 | 9 - 10.5 |
| Co | balance | balance | balance | balance | balance |

Microscopic Inspection for Microcleanliness of the alloy

The microscopic inspection for microcleanliness of the inventive alloy (example 1 and example 1a with an Ag core) and of the comparative alloy (example 2 and example 2a with an Ag core) was carried out according to the procedure and test method described above. Of 4 rods with an outer diameter of 31.75 mm, 5 transverse and 5 longitudinal sections were taken according to table 1 and metallographically prepared. The sections included a continuous plane from two surface locations and through the approximated center of the bar. The metallographically prepared sections were examined in the as-polished condition by scanning electron microscopy (SEM) using backscattered electron imaging (BEI). In BEI, the brightness of sample features is proportional to the atomic weight of the elements constituting those features.

Thus, in BEI, present inclusions consisting of heavier elements than the surrounding matrix material appear brighter than the matrix material. Inclusions consisting of lighter elements than the surrounding matrix material appear darker than the matrix material. Since nonmetallic inclusions (e.g. oxide or nitride inclusions) consist of lighter elements than the alloys of example 1 and example 2, in BEI these ceramic inclusions appear darker than the surrounding matrix material. Images were acquired at a magnification of 500X along a diametral line extending across the entire bar. Analysis of features darker and brighter than the background was conducted on the images using image analysis software to determine the maximum dimension for each detected feature. The largest dimension and area were recorded for each individual feature. The inclusions were categorized by largest dimension into 1 $\mu$m groups up to 14 $\mu$m. The total area of the dark and bright features was also calculated. Inclusions greater than 14 $\mu$m were also counted. Features smaller than 3.0 $\mu$m were not included in the measurements.

For each section, forty-eight fields of view were evaluated. For each direction, longitudinal and transverse, 480 images with a total area of 22.6 mm$^2$ were evaluated. The samples contained features that appeared darker and brighter than the bulk material using backscattered electron imaging. The darker features have a lower mean atomic number than the background and the brighter features have a higher mean atomic number than the background.

Results of the inclusion analysis of example 1 are shown in tables 4-6. Results of the inclusion analysis of example 2 are shown in tables 7-10. Image fields showing typical dark (ceramic) inclusions are shown in Figures 4,5, 7-10.

Alloy of the present invention (example 1):

## TABLE 4 - FEATURE COUNT TOTALS / EXAMPLE 1

| | | Number of Features | | | |
|---|---|---|---|---|---|
| Largest Dimension | | Longitudinal | | Transverse | |
| [$\mu$m] | | Dark | Bright | Dark | Bright |
| 3.0 | - 3.9 | 15 | 0 | 14 | 0 |
| 4.0 | - 4.9 | 4 | 0 | 2 | 0 |
| 5.0 | - 5.9 | 2 | 0 | 1 | 0 |
| 6.0 | - 6.9 | 0 | 0 | 0 | 0 |
| 7.0 | - 7.9 | 0 | 0 | 0 | 0 |
| 8.0 | - 8.9 | 0 | 0 | 0 | 0 |
| 9.0 | - 9.9 | 0 | 0 | 0 | 0 |
| 10.0 | - 10.9 | 0 | 0 | 0 | 0 |
| 11.0 | - 11.9 | 0 | 0 | 0 | 0 |
| 12.0 | - 12.9 | 0 | 0 | 0 | 0 |
| 13.0 | - 13.9 | 0 | 0 | 0 | 0 |
| 14.0 | - 14.9 | 0 | 0 | 0 | 0 |
| >14.9 | | 0 | 0 | 0 | 0 |
| | | | | | |
| Total | | 21 | 0 | 17 | 0 |

**TABLE 5 - TOTAL INCLUSION AREA MEASUREMENTS FOR EXAMPLE 1**

| | Area of Inclusions > 3 μm in Length for Examination Region | | | | | |
|---|---|---|---|---|---|---|
| | Darker | | Brighter | | All | |
| Sample | Total | Percent of Total Area | Total | Percent of Total Area | Total | Percent of Total Area |
| | [μm²] | [%] | [μm²] | [%] | [μm²] | [%] |
| Longitudinal | 121 | 0.0006 | 0 | 0.0000 | 121 | 0.0006 |
| Transverse | 97 | 0.0005 | 0 | 0.0000 | 97 | 0.0005 |

**TABLE 6 - LONGEST DARK FEATURES FOR EXAMPLE 1**

| | Feature Dimensions, [μm] | | |
|---|---|---|---|
| Number | Length | Breadth | Direction |
| 1 | 5.6 | 3.0 | Longitudinal |
| 2 | 5.4 | 3.7 | Longitudinal |
| 3 | 5.4 | 2.9 | Longitudinal |
| 4 | 4.9 | 2.2 | Longitudinal |
| 5 | 4.7 | 3.6 | Longitudinal |
| 6 | 4.6 | 1.9 | Longitudinal |
| 7 | 4.5 | 2.7 | Longitudinal |
| 8 | 4.5 | 2.4 | Longitudinal |
| 9 | 4.1 | 2.4 | Longitudinal |
| 10 | 3.9 | 3.0 | Longitudinal |

example 2 (comparative):

**TABLE 7 - FEATURE COUNT TOTALS / BARS 1-10 / ALL SAMPLES**

| | Number of Features | | | |
|---|---|---|---|---|
| Largest Dimension | Longitudinal | | Transverse | |
| [μm] | Dark | Bright | Dark | Bright |
| 3.0      - 3.9 | 25 | 21 | 6 | 46 |
| 4.0      - 4.9 | 19 | 7 | 3 | 11 |
| 5.0      - 5.9 | 7 | 1 | 1 | 1 |
| 6.0      - 6.9 | 6 | - | - | 1 |
| 7.0      - 7.9 | 7 | - | - | - |
| 8.0      - 8.9 | 4 | - | - | - |
| 9.0      - 9.9 | 1 | - | - | - |
| 10.0      - 10.9 | 2 | - | - | - |
| 11.0      - 11.9 | 2 | - | - | - |
| 12.0      - 12.9 | - | - | - | - |
| 13.0      - 13.9 | - | - | - | - |

(continued)

| Largest Dimension [μm] | Number of Features | | | |
| | Longitudinal | | Transverse | |
| | Dark | Bright | Dark | Bright |
|---|---|---|---|---|
| 14.0 - 14.9 | - | - | - | - |
| >14.9 | 4 | - | - | - |
| | | | | |
| Total | 77 | 29 | 10 | 59 |

**TABLE 8 - TOTAL INCLUSION AREA MEASUREMENTS FOR EXAMPLE 2**

| Sample | Area of Inclusions > 3 μm in Length for Examination Region | | | | | |
| | Darker | | Brighter | | All | |
| | Total [μm$^2$] | Percent of Total Area [%] | Total [μm$^2$] | Percent of Total Area [%] | Total [μm$^2$] | Percent of Total Area [%] |
|---|---|---|---|---|---|---|
| Longitudinal | 409 | 0.0018 | 75 | 0.0003 | 484 | 0.0021 |
| Transverse | 69 | 0.0003 | 152 | 0.0007 | 221 | 0.0010 |

**TABLE 9 - LONGEST DARK FEATURES FOR EXAMPLE 2**

| Number | Feature Dimensions, [μm] | | |
| | Length | Breadth | Direction |
|---|---|---|---|
| 1 | 33.4 | 1.9 | Longitudinal |
| 2 | 18.9 | 1.6 | Longitudinal |
| 3 | 17.8 | 2.3 | Longitudinal |
| 4 | 15.4 | 1.4 | Longitudinal |
| 5 | 11.8 | 1.0 | Longitudinal |
| 6 | 11.1 | 1.1 | Longitudinal |
| 7 | 10.6 | 1.0 | Longitudinal |
| 8 | 10.3 | 1.8 | Longitudinal |
| 9 | 9.5 | 1.5 | Longitudinal |
| 10 | 8.9 | 2.2 | Longitudinal |

**TABLE 10 - LONGEST BRIGHT FEATURES FOR EXAMPLE 2**

| Number | Feature Dimensions, [μm] | | |
| | Length | Breadth | Direction |
|---|---|---|---|
| 1 | 6.0 | 1.6 | Transverse |
| 2 | 5.6 | 2.8 | Longitudinal |
| 3 | 5.1 | 1.8 | Transverse |
| 4 | 4.9 | 2.3 | Transverse |
| 5 | 1.9 | 1.8 | Transverse |

(continued)

|  | Feature Dimensions, [$\mu$m] | | |
|---|---|---|---|
| Number | Length | Breadth | Direction |
| 6 | 1.0 | 1.8 | Longitudinal |
| 7 | 4.6 | 1.3 | Transverse |
| 8 | 4.5 | 1.2 | Transverse |
| 9 | 4.4 | 1.6 | Longitudinal |
| 10 | 4.4 | 0.9 | Transverse |

According to Table 8 of example 2 (comparative), the total area of dark inclusions found is 478 $\mu$m$^2$ (409 $\mu$m$^2$ in longitudinal direction and 69 $\mu$m$^2$ in transverse direction). According to Table 5 of example 1 (inventive), the total area of dark inclusions found is only 218 $\mu$m$^2$ (121 $\mu$m$^2$ in longitudinal direction and 97 $\mu$m$^2$ in transverse direction). So the amount of dark inclusions (Percent of total area) in example 1 (inventive) is only 4.8 ppm (0.00048 %) while in example 2 (comparative) the amount of dark inclusions is 11 ppm (0.0011 %). In terms of inclusions (micro-cleanliness) this means that example 1 (inventive) is more than 2 times cleaner than example 2 (comparative).

Fatigue Test Results

Two lots of wire of example 1 (dia. 102 $\mu$m) were tested against two lots of wire or example 2 (same diameter - 102 $\mu$m) having comparable mechanical properties (UTS of 1862 - 1875 MPa).

**Table 11**

| Material | Example 1 (inventive) | | Example 2 (comparative) | |
|---|---|---|---|---|
| Batch | Lot A | Lot B | Lot C (Fig. 5 & 6) | Lot D |
| UTS [MPa] | 1870 | 1875 | 1862 | 1871 |
| YM [GPa] | 190 | 191 | 190 | 190 |
| Elongation [%] | 2.8 | 2.9 | 2.7 | 2.8 |

At an applied stress of 700 MPa, the wire of all four lots reached the fatigue endurance limit, means the wire does not fail and tests are stopped after 100 Million cycles. While the wire of example 1 showed no outliers at 700 MPa and below, 4 samples of example 2 failed at less than 2.7 Million cycles and two other samples ran 40 - 50 Million cycles. All other samples tested at an applied stress of 700 MPa and below survived 100 Million cycles without rupture. For Example 2 wire lot C, sample C25 tested at an applied stress of 700 MPa broke after only 71,790 cycles and sample C31 tested at an applied stress of 520 MPa broke after only 145,260 cycles. Sample C26 tested at an applied stress of 700 MPa broke after 47,547,540 cycles and sample C29 tested at an applied stress of 700 MPa broke after 41,282,990 cycles. For example 2 wire lot D, sample D27 tested at an applied stress of 700 MPa broke after only 549,227 cycles and sample D35 tested at an applied stress of 520 MPa broke after only 2,689,952 cycles. SEM-images of sample C25 shows an inclusion at the fracture surface. In EDX analysis, high peaks for Aluminium, Magnesium, Chromium and Oxygen were found. This mixed-oxide inclusion was identified as the crack initiation point for the early failure of this sample. An SEM-image of sample D35 also shows an inclusion at the fracture surface. Again, in EDX analysis, high peaks for Aluminium, Magnesium, Chromium and Oxygen were found. Also this mixed-oxide inclusion can be identified as the crack initiation point for the early failure of this sample. SEM investigations of samples C31 and D27 also showed oxide-inclusions at the fracture surface which were identified causing the early failure. For both samples, the same elements (Aluminium, Magnesium, Chromium, Oxygen) show high peaks in EDX analysis for these two samples.

**Table 12**

| stress level [MPa] | Example 1 (inventive) (Lot A) | Example 1 (inventive) (Lot B) | Example 2 (comparative) (Lot C) | Example 2 (comparative) (Lot D) |
|---|---|---|---|---|
| | No. of cycles | No. of cycles | No. of cycles | No. of cycles |
| 1430 | 21092 | 66720 | 20700 | 35130 |
| 1430 | 12740 | 63781 | 22140 | 57120 |
| 1430 | 28919 | 59140 | 36120 | 32460 |
| 1430 | 19334 | 37942 | 23550 | 35010 |
| 1430 | 18119 | 39178 | 18270 | 28996 |
| 1430 | 21983 | 22380 | 18150 | 33313 |
| 1040 | 38670 | 128644 | 136680 | 149971 |
| 1040 | 58474 | 218106 | 1004289 | 308580 |
| 1040 | 46980 | 194108 | 8656363 | 80766 |
| 1040 | 34806 | 41310 | 107640 | 178770 |
| 1040 | 38045 | 539089 | 7856526 | 74323 |
| 1040 | 39120 | 290101 | 6852041 | 127650 |
| 880 | 5888420 | 4715690 | 39485962 | 72523366 |
| 880 | 4233055 | 5816670 | 102020453 | 100000000 |
| 880 | 6324748 | 2144125 | 100800000 | 6171230 |
| 880 | 13571905 | 2068519 | 114000000 | 4407264 |
| 880 | 8824316 | 1725656 | 101000000 | 102000000 |
| 880 | 7680042 | 1815390 | 100000000 | 57462763 |
| 800 | 104700000 | 40051186 | - | - |
| 800 | 96874223 | 16938278 | - | - |
| 800 | 59716187 | 26518613 | - | - |
| 800 | 54411683 | 79084889 | - | - |
| 800 | 64971417 | 46467823 | - | - |
| 800 | 100000000 | 24231864 | - | - |
| 700 | 103000000 | 105000000 | 71790 | 100000000 |
| 700 | 109852488 | 97119288 | 47547540 | 100000000 |
| 700 | 101589761 | 107000000 | 100000000 | 549227 |
| 700 | 101623566 | 104000000 | 100000000 | 101337563 |
| 700 | 102000000 | 101000000 | 41282990 | 100000000 |
| 700 | 103000000 | 103000000 | 100000000 | 100000000 |
| 520 | 100000000 | 100000000 | 145260 | 100000000 |
| 520 | 118987000 | 106000000 | 110800000 | 110800000 |
| 520 | 102064330 | 102000000 | 101000000 | 101000000 |
| 520 | 100963860 | 100613526 | 100500000 | 100500000 |
| 520 | 100845911 | 100845911 | 108000000 | 2689952 |
| 520 | 101009712 | 101006089 | 100000000 | 112000000 |

These fatigue test results are plotted in Figure 12. As can be seen from this plot, lots C and D (comparative) show significantly more undesired outliers that for lots A and B (inventive).

Three lots of example 1a/Ag28% wire (diameter 127 μm) were also tested against three lots of example 2a wire (same diameter - 127 μm). All six wire lots have comparable mechanical properties (UTS of 1456 - 1475 MPa).

**Table 13**

| - | Example 1a + 28 wt. % Ag (inventive) | | | Example 2a + 28 wt. % Ag (comparative) | | |
|---|---|---|---|---|---|---|
| Batch | Lot E | Lot F | Lot G | LotH | Lot J (Fig. 10 & 11) | Lot K |
| UTS [MPa] | 1456 | 1469 | 1474 | 1460 | 1462 | 1475 |
| YM [GPa] | 121 | 121 | 122 | 121 | 122 | 122 |
| Elongation [%] | 2.2 | 2.3 | 2.3 | 2.1 | 2.0 | 2.3 |

At an applied stress of 414 MPa, the wire of all four lots reached the fatigue endurance limit, means the wire does not fail and tests are stopped after 100 Million cycles. While example 1a /Ag28% wire showed no outliers at 414 MPa and below, 4 samples of example 2a /Ag28% wire failed at less than 1.4 Million cycles. All other samples tested at an applied stress of 414 MPa and below survived 100 Million cycles without rupture. For example 2a /Ag28% wire lot H, sample H24 tested at an applied stress of 414 MPa broke after only 1,041,679 cycles. Sample J18 tested at an applied stress of 518 MPa broke after 588,028 cycles and sample J23 tested at an applied stress of 414 MPa broke after 263,488 cycles. Sample K24 tested at an applied stress of 414 MPa broke after 1,355,189 cycles. As an example, SEM-images of sample J23 show an inclusion at the fracture surface. In EDX analysis, high peaks for Aluminium, Magnesium, Chromium and Oxygen were found. This mixed-oxide inclusion was identified as the crack initiation point for the early failure of this sample. SEM investigations of samples H24, J18 and K24 also showed oxide-inclusions at the fracture surface which were identified causing the early failure. For all three samples, the same elements (Aluminium, Magnesium, Chromium, Oxygen) show high peaks in EDX analysis for these three samples.

**Table 14**

| | Example 1a + 28 wt. % Ag | | | Example 2a + 28 wt. % Ag | | |
|---|---|---|---|---|---|---|
| | lot E | lot F | lot G | lot H | lot J | lot K |
| stress level [MPa] | No. of cycles | No. of cycles | No. of cycles | No. of cycles | No. of cycles | No. of cycles |
| 969 | 31,887 | 33,065 | 21,487 | 47,982 | 38,148 | 32,926 |
| 969 | 29,321 | 28,116 | 23,461 | 43,661 | 41,085 | 20,818 |
| 969 | 32,555 | 29,941 | 28,763 | 30,298 | 33,187 | 32,299 |
| 969 | 26,918 | 23,418 | 18,464 | 37,888 | 36,247 | 38,901 |
| 969 | 22,089 | 30,467 | 20,198 | 43,092 | 41,944 | 42,978 |
| 725 | 246,766 | 231,412 | 114,746 | 74,414 | 235,494 | 109,597 |
| 725 | 199,054 | 189,441 | 123,746 | 79,498 | 128,377 | 92,419 |
| 725 | 287,665 | 262,994 | 168,374 | 94,638 | 118,922 | 91,877 |
| 725 | 200,822 | 186,242 | 145,355 | 75,062 | 162,522 | 102,834 |
| 725 | 500,045 | 290,377 | 169,176 | 62,082 | 238,611 | 99,864 |
| 580 | 1,405,296 | 1,612,743 | 979,651 | 409,644 | 6,780,968 | 311,974 |
| 580 | 1,077,510 | 1,131,168 | 1,846,396 | 668,132 | 12,545,505 | 8,369,715 |
| 580 | 8,201,513 | 993,416 | 1,684,673 | 1,031,447 | 1,945,002 | 3,001,478 |
| stress level [MPa] | No. of cycles | No. of cycles | No. of cycles | No. of cycles | No. of cycles | stress level [MPa] |
| 580 | 2,511,763 | 2,197,173 | 639,464 | 342,282 | 2,639,912 | 219,634 |
| 580 | 841,436 | 884,196 | 2,076,465 | 3,539,353 | 8,566,249 | 2,009,899 |
| 518 | 40,051,186 | 86,414,732 | 71,763,385 | 100,000,000 | 100,000,000 | 100,000,000 |

(continued)

| stress level [MPa] | No. of cycles | No. of cycles | No. of cycles | No. of cycles | No. of cycles | stress level [MPa] |
|---|---|---|---|---|---|---|
| 518 | 100,000,000 | 78,411,674 | 83,944,821 | 100,000,000 | 100,000,000 | 100,000,000 |
| 518 | 79,084,889 | 100,000,000 | 35,946,337 | 100,000,000 | 588,028 | 100,000,000 |
| 518 | 46,467,823 | 100,000,000 | 100,000,000 | 100,000,000 | 100,000,000 | 100,000,000 |
| 518 | 100,000,000 | 87,867,423 | 54,676,179 | 100,000,000 | 100,000,000 | 100,000,000 |
| 414 | 100,000,000 | 100,000,000 | 100,000,000 | 100,000,000 | 100,000,000 | 100,000,000 |
| 414 | 100,000,000 | 100,000,000 | 100,000,000 | 100,000,000 | 100,000,000 | 100,000,000 |
| 414 | 100,000,000 | 100,000,000 | 98,674,345 | 100,000,000 | 263,488 | 100,000,000 |
| 414 | 100,000,000 | 100,000,000 | 100,000,000 | 1,041,679 | 100,000,000 | 1,355,189 |
| 414 | 100,000,000 | 100,000,000 | 96,674,523 | 100,000,000 | 100,000,000 | 100,000,000 |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |

These fatigue test results are plotted in Figure 13. As can be seen from this plot, lots H, J and K (comparative) show significantly more undesired outliers that for lots E, F and G (inventive).

Pacemaker lead

A wire with thickness 25 $\mu$m was prepared according to the method described above and with compositions of the alloy as given in table 3. The wires were arranged into a lead as described in figure 1. The leads were tested for fatigue resistance and for impurity inclusions. The results are shown in table 15.

**Table 15**

| Example | Fatigue resistance | Purity from inclusions |
|---|---|---|
| Heat 1 | ++ | ++ |
| Heat 2 | ++ | ++ |
| Heat 3 | ++ | ++ |
| Commercial heat | - | - |
| ++ = very good, - = poor | | |

Reference list

10 Wire
20 First region
30 Further region
50 Pacemaker
60 Electrode unit
70 Electronic unit
100 Cable
110 First mass
120 Body

130     Precursor
140     Lead/Cable bundle
C       chuck to bushing distance
H       height of loop
R       minimum of radius of curvature
P       bushing load or lateral force at the chuck
#A1     Dark inclusion

**Claims**

1.  A method for manufacturing a cable (100), comprising the steps of:

    - providing several raw wires made of a wire material,
    - drawing the raw wires into wires (10),
    - coiling the wires (10) into a cable (100), and
    - heat treating the cable (100),

    wherein the wire material comprises an alloy comprising the following alloy components:

    a) Cr in the range from about 10 to about 30 wt. %;
    b) Ni in the range from about 20 to about 50 wt. %;
    c) Mo in the range from about 2 to about 20 wt. %;
    d) Co in the range from about 10 to about 50 wt. %;

    wherein the Al content of the alloy is less than about 0.01 wt. %; and
    wherein each wt. % is based on the total weight of the alloy.

2.  Method according to claim 1, wherein the Cr, Ni, Mo and Co components are major constituents of the alloy with at least about 95 wt. % of the alloy being Cr, Ni, Mo and Co.

3.  Method according to one of the preceding claims, wherein the raw wires are drawn with a cold work percentage in a range of 98 and 99 %.

4.  Method according to claim 1 or 2, wherein the raw wires are drawn with a cold work percentage in a range of 80 and 86 %.

5.  Method according to one of the preceding claims, wherein the heat treatment of the cable (100) is in a range of 480 to 750 °C with a dwell time of 5 to 6 seconds.

6.  Method according to one of the preceding claims, wherein the heat treatment of the cable (100) is a stress relief treatment in a temperature range of 480 to 580 °C to improve a low cycle fatigue performance of the cable (100).

7.  Method according to one of the claims 1 to 5, wherein the heat treatment of the cable (100) is an annealing in a temperature range of 780 to 810 °C to improve a high cycle fatigue performance of the cable (100).

8.  Method according to one of the preceding claims, further comprising an initial drawing of a raw material into the raw wires.

9.  Method according to the preceding claim, wherein the raw material is drawn with a cold work percentage in a range of 95 and 96 %.

10. Method according to one of the preceding claims, further comprising an initial heat treatment of the raw wires before the drawing of the raw wires into the wires (10).

11. Method according to the preceding claim, wherein the initial heat treatment of the raw wires is an annealing in a temperature range of 875 to 1100 °C.

12. Method according to one of the preceding claims, wherein the wire material further comprises an additional material different to the Cr, Ni, Mo and Co alloy, and wherein the additional material forms a core and the Cr, Ni, Mo and Co alloy forms a shell around the core when the wire is seen in a cross section.

13. Method according to one of the claim 1 to 11, wherein the wire material further comprises an additional material different to the Cr, Ni, Mo and Co alloy, and wherein the Cr, Ni, Mo and Co alloy forms a core and the additional material forms a shell around the core when the wire is seen in a cross section.

14. A cable (100) comprising drawn, coiled and heat treated wires (10) made of a wire material, wherein the wire material comprises an alloy comprising the following alloy components:

  a) Cr in the range from about 10 to about 30 wt. %;
  b) Ni in the range from about 20 to about 50 wt. %;
  c) Mo in the range from about 2 to about 20 wt. %;
  d) Co in the range from about 10 to about 50 wt. %;

wherein the A1 content of the alloy is less than about 0.01 wt. %; and
wherein each wt. % is based on the total weight of the alloy.

15. A medical device (50) comprising a cable (100) according to the preceding claim as a lead (140).

Fig. 1

Fig. 2

Fig. 3

Fig. 4

Fig. 5

Fig. 6

Fig. 7

Fig. 8

Fig. 9

Fig. 10

Fig. 11

Intensity

Fig. 12

Fig. 13

Fig. 14

Fig. 15

Fig. 16

Fig. 17

Fig. 18

Europäisches
Patentamt

European
Patent Office

Office européen
des brevets

**EUROPEAN SEARCH REPORT**

Application Number

EP 17 17 5928

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/075168 A1 (SCHAFFER JEREMY E [US]) 25 March 2010 (2010-03-25) * abstract; figure 29 * * paragraph [0002] - paragraph [0003] * * paragraph [0008] * * paragraph [0092] * * paragraph [0100] * * paragraph [0103] - paragraph [0000] * * paragraph [0192] - paragraph [0199] * * paragraph [0284] - paragraph [0290] * ----- | 1-11,14, 15 | INV. A61N1/05 H01B1/02 |
| A,D | US 2005/051243 A1 (FORBES JONES ROBIN M [US] ET AL) 10 March 2005 (2005-03-10) * the whole document * ----- | 1-15 | |
| A | US 2010/206612 A1 (SCHIEFER HERWIG [DE]) 19 August 2010 (2010-08-19) * the whole document * ----- | 1-15 | |
| A | US 2004/267107 A1 (LESSAR JOSEPH F [US] ET AL) 30 December 2004 (2004-12-30) * the whole document * ----- | 1-15 | |

TECHNICAL FIELDS
SEARCHED      (IPC)

A61N
H01B

The present search report has been drawn up for all claims

| Place of search | Date of completion of the search | Examiner |
|---|---|---|
| Munich | 11 December 2017 | Molina Silvestre, A |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

.........................................................................

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 17 17 5928

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2017

| Patent document cited in search report | | Publication date | Patent family member(s) | | Publication date |
|---|---|---|---|---|---|
| US 2010075168 | A1 | 25-03-2010 | EP | 2334838 A1 | 22-06-2011 |
| | | | JP | 5927405 B2 | 01-06-2016 |
| | | | JP | 6094901 B2 | 15-03-2017 |
| | | | JP | 2012502190 A | 26-01-2012 |
| | | | JP | 2015061944 A | 02-04-2015 |
| | | | US | 2010075168 A1 | 25-03-2010 |
| | | | US | 2014378949 A1 | 25-12-2014 |
| | | | WO | 2010033873 A1 | 25-03-2010 |
| US 2005051243 | A1 | 10-03-2005 | AT | 554192 T | 15-05-2012 |
| | | | CN | 1867687 A | 22-11-2006 |
| | | | DK | 1664360 T3 | 14-05-2012 |
| | | | EP | 1664360 A1 | 07-06-2006 |
| | | | HK | 1092842 A1 | 11-01-2013 |
| | | | JP | 5165241 B2 | 21-03-2013 |
| | | | JP | 2007504362 A | 01-03-2007 |
| | | | US | 2005051243 A1 | 10-03-2005 |
| | | | WO | 2005026399 A1 | 24-03-2005 |
| US 2010206612 | A1 | 19-08-2010 | DE | 102009009558 A1 | 26-08-2010 |
| | | | US | 2010206612 A1 | 19-08-2010 |
| US 2004267107 | A1 | 30-12-2004 | NONE | | |

EPO FORM P0459

For more details about this annex : see Official Journal of the European Patent Office, No. 12/82

**REFERENCES CITED IN THE DESCRIPTION**

**Patent documents cited in the description**

- WO 2005026399 A1

- US 20050051243 A1